# EUROPEAN PATENT APPLICATION

(11) **EP 4 523 623 A1**
(43) Date of publication of application: **19.03.2025**
(21) Application number: 22946047.2
(22) Date of filing: 14.10.2022
(51) Int. Cl.: A61B 5/151, A61M 5/158

(54) **NEEDLE INSERTION MECHANISM, INJECTION CHANNEL MODULE, AND DRUG INJECTION SYSTEM**

(60) Divisional application: 24221497.1
(71) Applicant: CC Biotechnology Corporation, Tainan City, Taiwan 70955 (TW)
(72) Inventor: YEH, Chin-Min, Tainan City (TW); WANG, Chih-Wei, Tainan City (TW)
(74) Representative: Cabinet Chaillot
(86) International application number: PCT/CN2022/125386
(87) International publication number: WO 2024/077599

(57) **Abstract**

The present invention relates to a needle output mechanism (10), an injection channel module (1) and a drug injection system. The injection channel module (1) includes a needle output mechanism (10) and an adapting mechanism (20). The drug injection system includes an injection channel module (1), a drug storage module (2) detachably connected to the injection channel and a driving module (3). The needle output mechanism (10) includes a casing (11), and a needle output operation assembly (12) and a piercing assembly (13) mounted in the casing (11). Wherein, a needle seat (124) is driven by a rotating element (122) to complete movement of making the piercing assembly (13) that is formed by combining a piercing needle (131) and a conduit element (132) to output needle and then the piercing needle (131) is retracted and the conduit element (132) is detained to protrude out of the casing (11).

## Description

### Field of the Invention

The present invention relates to the technical field of drug injection, especially to a needle output mechanism, an injection channel module, and a drug injection system.

### Description of the Prior Art(s)

A conventional drug injection system for subcutaneous injection of medicinal liquid into human body mainly uses an injection mechanism that is able to be operated manually or electrically to connect a drug container, so as to insert a needle set of the injection mechanism into the human body to leave a soft needle of the needle set pierced through skin of the human body and withdraw a hard needle. The medicinal liquid is then injected manually or electrically into subcutaneous tissue of the human body through the soft needle that pierce into the human body.

The afore-mentioned injection mechanism has a composite needle set including a soft needle and a hard needle to provide components for subcutaneous injection in the human body. However, the conventional injection mechanism is operated by a complex needle output operating mechanism, which causes the needle set to pierce into the human body with the soft needle sleeved around the hard needle and then the hard needle is withdrawn, leaving the soft needle in the human body. The operation of the needle set is relatively cumbersome.

In addition, based on the afore-mentioned problems of the conventional injection mechanism, after an adapting mechanism that is assembled with the injection mechanism is connected to a drug storage mechanism, delivery path of the medicinal liquid is also relatively complicated. Moreover, combination structure of the mechanisms in the drug injection system is also difficult to provide an ideal injection method. It is necessary to further optimize a needle output mechanism in the drug injection system.

### Content of the Invention

The technical problem to be solved by this invention is to provide a needle output mechanism, an injection channel module and a drug injection system to solve the problem that the needle output mechanism in the conventional drug injection system uses a complex needle output mechanism, and causes an operation method of a piercing assembly, which is performed by piercing into a human body in the form of a conduit element sleeving on a piercing needle and then leaving the conduit element on the human body after withdrawing the piercing needle, being cumbersome.

The technical solution proposed by the present invention is to provide a needle output mechanism including:
a casing;
a needle output operation assembly assembled in the casing and the needle output operation assembly including a needle output element, a rotating element and a needle seat; the needle output element movably mounted in the casing; the rotating element rotatably mounted in the casing and the rotating element being able to be driven by the needle output element to rotate; the needle seat movably mounted in the rotating element and the needle seat being able to be driven by the rotating element to move be between a first position and a second position; and
a piercing assembly assembled in the casing and the piercing assembly including a piercing needle and a conduit element; the piercing piercing assembly connected with the needle seat; the conduit element being able to be combined with the piercing needle; wherein in a needle output state, the piercing assembly that is formed by combining the conduit element and the piercing needle is driven by the needle output operation assembly to cause at least part of the piercing assembly to protrude out of the casing; wherein in a needle retraction state, the piercing needle moves upward along with the needle seat to retract the piercing needle.

In the needle output mechanism as described: the needle output element is mounted in the casing to move linearly; the rotating element is mounted in the casing and is able to be driven by the needle output element to rotate forwardly or reversely.

In the needle output mechanism as described: the needle output operation assembly further includes a needle seat plug and the needle seat plug is mounted in the casing; the piercing needle protrudes into the needle seat plug; the conduit element is disposed inside the needle seat plug and is sleeved around the piercing needle; the needle seat is able to be driven by the rotating element to move between the rotating element and the needle seat plug.

In the needle output mechanism as described: the needle seat plug has an accommodating room, a liquid injection room disposed at a lower end of the accommodating room, a connecting port disposed on a side of the liquid injection room and communicating with the liquid injection room, and a sealed portion disposed at a lower end of the liquid injection room and exposed on a back surface of the casing; wherein in the needle output state, the piercing assembly pierces through the sealed portion on a bottom of the needle seat plug; wherein in the needle retraction state, part of the conduit element protrudes out of the sealed portion.

In the needle output mechanism as described: the rotating element has a threaded hole formed therein; a threaded portion is provided on the needle seat and engages with the threaded hole of the rotating element; a gear portion is provided on an upper end of the rotating element; the needle output element has an elongated rail groove and a toothed driving portion provided in the elongated rail groove, and a length of the elongated rail groove is defined as an operation stroke; the toothed driving portion includes a forward rotating rack segment and a reverse rotating rack segment; the forward rotating rack segment and the reverse rotating rack segment are two different sections respectively disposed along the elongated rail groove and are disposed on two opposite sides; the gear portion of the rotating element is disposed in the elongated rail groove; wherein with movement of the needle output element along the operation stroke, the gear portion of the rotating element is able to mesh with either the forward rotating rack segment or the reverse rotating rack segment respectively, such that the rotating element is able to be driven to rotate in a forward direction and in a reverse direction.

In the needle output mechanism as described: the casing includes a base and an outer casing sleeved around the base; a back side of the outer casing is mounted with a contact end cap that is able to travel within a movement stroke; a resilient element is disposed between the outer casing and the contact end cap; the needle output element is mounted in the base and is able to move within a distance of an operation stroke; the needle output element is able to be locked in the base at a starting position and an end position of the operation stroke respectively to be in a locked state; resilient force of the resilient element acts on the contact end cap, causing part of the contact end cap to protrude from a back side of the outer casing; the contact end cap is able to be forced to enter the outer casing to release the needle output element that is located at the starting position of the operation stroke from the locked state.

In the needle output mechanism as described: the base has a first engaging hole disposed on the starting position of the operation stroke and a second engaging hole disposed on the end position of the operation stroke; an engaging hook is provided on a rear end of the needle output element and the engaging hook is able to selectively engage in the first engaging hole or the second engaging hole; the contact end cap has an unlocking pushing portion; wherein when the contact end cap is forced to enter the outer casing, the unlocking pushing portion is able to push the engaging hook of the needle output element to disengage from the first engaging hole to release the needle output element that is locked from the locked state.

With the overall structure of the needle output mechanism described above, the needle output mechanism mainly uses the combined structure of the needle output element in the piercing assembly with the rotating element and the needle seat to allow the needle output element to drive the rotating element to first rotate forward and then reverse in one operation stroke. Moreover, the needle seat of the needle seat set is driven by the rotating element to make the piercing assembly that is formed by combining the conduit element and the piercing needle to output needle. Then the piercing needle moves upward along with the needle seat to retract the piercing needle, and the conduit element remains protruding out of the casing, which is in a needle output state. In this way, operation of the needle output mechanism when outputting the needle is made easier, thereby achieving the inventive purpose of optimizing the product.

The casing of the needle output mechanism of the present invention may further include a base and an outer casing sleeved around the base. A back side of the outer casing is mounted with a contact end cap that is able to travel within a movement stroke. A resilient element is disposed between the outer casing and the contact end cap. The needle output element is mounted in the base and is able to move within a distance of an operation stroke. The needle output element is able to be locked in the base at a starting position and an end position of the operation stroke respectively to be in a locked state. Resilient force of the resilient element acts on the contact end cap, causing part of the contact end cap to protrude from a back side of the outer casing. The contact end cap is able to be forced to enter the outer casing to release the needle output element that is located at the starting position of the operation stroke from the locked state. Thus, a security protection mechanism is provided in the needle output mechanism, so as to ensure that that the needle output element does not accidentally output the needle when the needle output mechanism is not in use. After injection, the contact end cap may be sleeved around the piercing assembly, so as to prevent the piercing assembly from exposing and stabbing someone.

The technical solution proposed by the present invention is to provide an injection channel module including:
a needle output mechanism as described above: and
an adapting mechanism assembled to a side of the needle output mechanism and the adapting mechanism including an adapting seat, a flow channel switching valve, a piston barrel and a piston rod; the adapting seat assembled in the needle output mechanism; the flow channel switching valve and the piston barrel both mounted in the adapting seat; the adapting seat having a medicinal liquid inflow channel and a medicinal liquid outflow channel that communicate with the piston barrel; the flow channel switching valve communicating with the connecting port of the needle output mechanism through the medicinal liquid outflow channel of the adapting seat; the piston rod linearly movably assembled in the piston barrel.

In the injection channel module as described: the flow channel switching valve includes a one-way input valve portion and a one-way output valve portion; two ends of the piston barrel are respectively a connecting end and a barrel opening end; the connecting end is connected to the flow channel switching valve and the barrel opening end protrudes out of the adapting seat; the piston barrel has a piston chamber formed therein; the connecting end has a medicinal liquid inlet and a medicinal liquid outlet communicating with the piston chamber; the medicinal liquid inlet is connected to the one-way input valve portion and the medicinal liquid outlet is connected to the one-way output valve portion; the piston rod is able to be driven to move linearly in the piston chamber of the piston barrel to draw medicinal liquid into the piston chamber through the medicinal liquid inflow channel and the one-way input valve portion in one way and output the medicinal liquid to the medicinal liquid outflow channel through the one-way output valve portion in one way.

With the overall structure of the injection channel module described above, in addition to optimize needle output movement of the needle output mechanism, the injection channel module also allows the adapting mechanism to be in direct connect with the drug storage module. Thus, the medicinal liquid can be directly delivered to the needle output mechanism through the adapting mechanism, such that the driving module connected externally to the adapting mechanism does not contact with the medicinal liquid to be injected at all during the injection process, reducing the possibility of contaminating the medicinal liquid.

In order to achieve the aforementioned objective, the present invention further provides a drug injection system comprising:
an injection channel module including a needle output mechanism as described above and an adapting mechanism, wherein the adapting mechanism is assembled to a side of the needle output mechanism and the adapting mechanism includes an adapting seat, a flow channel switching valve, a piston barrel and a piston rod; the adapting seat is assembled in the needle output mechanism; the flow channel switching valve and the piston barrel are both mounted in the adapting seat; the adapting seat has a medicinal liquid inflow channel and a medicinal liquid outflow channel that communicate with the piston barrel; the flow channel switching valve communicates with the connecting port of the needle output mechanism through the medicinal liquid outflow channel of the adapting seat; the piston rod is linearly movably assembled in the piston barrel;
a drug storage module including a medicinal liquid storage container and an outer covering; the medicinal liquid storage container including a container body filled with medicinal liquid and a container connector assembled to the container body; the container connector connected to the medicinal liquid inflow channel of the adapting mechanism of the injection channel module; the outer covering being able to be sleeved around the medicinal liquid storage container and connected with the casing of the needle output module; and
a driving module detachably assembled to the injection channel module and being able to drive the piston rod of the adapting mechanism to move.

In the drug injection system as described: the container connector includes a connector body; the connector body includes a container connecting end, an infusion connecting end and an infusion end portion; the infusion channel extends from the container connecting end to the infusion connecting end and is connected with the container body through the container connecting end; the infusion connecting end is connected to the medicinal liquid inflow channel of the adapting mechanism; the infusion end portion has an infusion branch channel communicating with the infusion channel and an infusion plug mounted in the infusion branch channel and sealing the infusion branch channel; the infusion plug has a plug end exposed outside the connector body and the plug end is exposed on a back surface of the outer covering.

In the drug injection system as described: the casing of the needle output mechanism includes a base and an outer casing sleeved around the base; a back side of the outer casing is mounted with a contact end cap that is able to travel within a movement stroke; a resilient element is disposed between the outer casing and the contact end cap; the needle output element is mounted in the base and is able to move within a distance of an operation stroke; the needle output element is able to be locked in the base at a starting position and an end position of the operation stroke respectively to be in a locked state; resilient force of the resilient element acts on the contact end cap, causing part of the contact end cap to protrude from a back side of the outer casing; the contact end cap is able to be forced to enter the outer casing to release the needle output element that is located at the starting position of the operation stroke from the locked state;
the flow channel switching valve includes a one-way input valve portion and a one-way output valve portion; two ends of the piston barrel are respectively a connecting end and a barrel opening end; the connecting end is connected to the flow channel switching valve and the barrel opening end protrudes out of the adapting seat; the piston barrel has a piston chamber formed therein; the connecting end has a medicinal liquid inlet and a medicinal liquid outlet communicating with the piston chamber; the medicinal liquid inlet is connected to the one-way input valve portion and the medicinal liquid outlet is connected to the one-way output valve portion; the piston rod is able to be driven to move linearly in the piston chamber of the piston barrel to draw medicinal liquid into the piston chamber through the medicinal liquid inflow channel and the one-way input valve portion in one way and output the medicinal liquid to the medicinal liquid outflow channel through the one-way output valve portion in one way;
the driving module includes a housing, a control unit, a motor with a central shaft, and a transmission assembly; the control unit, the motor and the transmission assembly are all mounted in the housing; the motor is electrically connected to and is controlled by the control unit; the transmission assembly includes a driving wheel, a connecting rod and a drive element; the driving wheel is assembled to the central shaft of the motor; an end of the connecting rod is eccentrically connected pivotally to the driving wheel and another end of the connecting rod is connected to the drive element and is detachably connected to a distal end of the piston rod through the drive element; a movement direction of the connecting rod and the drive element is parallel with a central axis of the piston rod.

In the drug injection system as described: the housing includes a housing body and a housing cover mounted on the housing body; a power supply unit and two electrical connectors are able to be mounted in the housing; the power supply unit is able to be electrically connected to the control unit through the two electrical connectors; an insulating sheet is provided between an end of the power supply unit and one of the electrical connectors that corresponds in position thereto for insulation and isolation; the insulating sheet protrudes out of the housing.

In the drug injection system as described: the driving module includes an activating switch, a confirming switch and a counting element; the activating switch, the confirming switch and the counting element are all electrically connected to the control unit; the activating switch is mounted on the housing and is disposed at the end position of the operation stroke of the needle output element; wherein when the needle output element is pressed to the end position, the activating switch is activated;
the confirming switch is mounted in the housing and is disposed at an end position of the movement stroke of the contact end cap; wherein when the contact end cap is pressed to the end position, the confirming switch is activated;
the counting element is mounted in the housing and is disposed at a movement path of the drive element; wherein when the drive element is moved, the counting element is activated.

With the overall structure of the drug injection system described above, in addition to that the injection channel module has the effect of optimizing the needle output movement of the needle output mechanism, the medicinal liquid in the drug container may be delivered to the piercing assembly to be injected by arrangement of liquid channels in the adapting mechanism, the adapting mechanism that is detachably connected to the medicinal liquid storage container of the drug storage module, and the piston rod of the adapting mechanism that is detachably connected to the driving module and is able to be driven by a driving mechanism. With the modular combination structure, the disposable drug injection system and the combination structure of the adapting mechanism externally connected to the driving module, the driving module is prevented from being in contact with the medicinal liquid during the injection process, such that the safety of the drug injection system in use can be ensured and the driving assembly also provides precise control over injection drive force and injection dose.

### Description of the Drawings

Fig. 1 is a schematic perspective view of an embodiment of a needle output mechanism of the present invention;
Fig. 2 is a schematic exploded perspective view of the embodiment of the needle output mechanism shown in Fig. 1;
Fig. 3 is a schematic top plane view of combination of a needle output element and a rotating element of the embodiment of the needle output mechanism shown in Fig. 2;
Fig. 4 is a schematic cross-sectional side view of the embodiment of the needle output mechanism shown in Figs. 1 to 3;
Fig. 5 is a schematic cross-sectional side view from another perspective of the embodiment of the needle output mechanism shown in Figs. 1 to 3;
Fig. 6 is a schematic perspective view of an embodiment of an injection channel module of the present invention;
Fig. 7 is a schematic exploded perspective view of the embodiment of the injection channel module shown in Fig. 6;
Fig. 8 is a schematic exploded perspective view of an adapting mechanism of the embodiment of the injection channel module shown in Fig. 7;

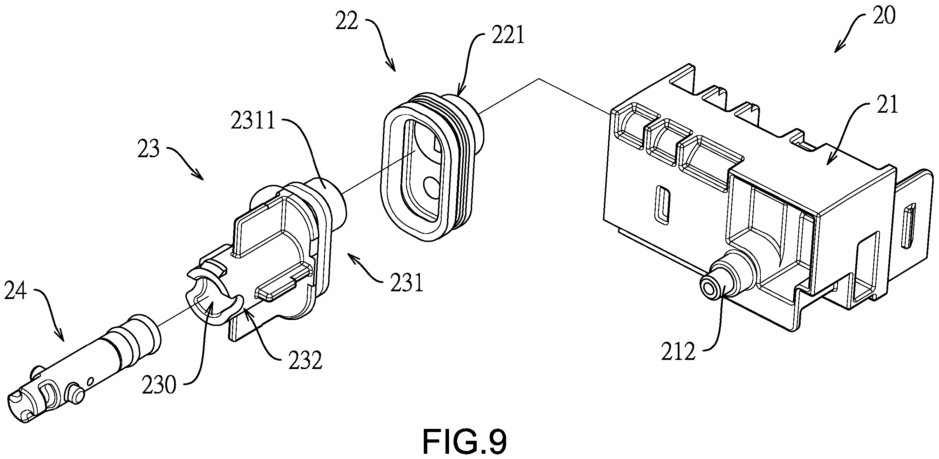

Fig. 9 is a schematic exploded perspective view from another perspective of an adapting mechanism of the embodiment of the injection channel module shown in Fig. 7;
Fig. 10 is a schematic partial cross-sectional view of the embodiment of the injection channel module shown in Fig. 6;
Fig. 11 is a schematic perspective view of an embodiment of a drug injection system of the present invention;
Fig. 12 is a schematic exploded perspective view of the embodiment of the drug injection system shown in Fig. 11;
Fig. 13 is a schematic exploded perspective view of a drug storage module of the embodiment of the drug injection system shown in Figs. 11 and 12;
Fig. 14 is a schematic partial cross-sectional view of combination of the drug storage module and the injection channel module shown in Figs. 11 to 13;
Fig. 15 is a schematic partial exploded perspective view of a driving module of the embodiment of the drug injection system shown in Figs. 11 and 12;
Fig. 16 is a schematic top plane view of the driving module shown in Fig. 15 with a housing cover being removed;
Fig. 17 is a schematic top plane view of the embodiment of the drug injection system shown in Figs. 11 and 12 with the housing cover and other parts being removed;
Fig. 18 is a schematic perspective view of a motor in combination with a transmission assembly of the driving module connecting with a piston rod of the adapting mechanism shown in Figs. 15 to 17;
Fig. 19 is a schematic operational perspective view of the motor in combination with the transmission assembly of the driving module connecting with the piston rod of the adapting mechanism shown in Figs. 15 to 17;
Fig. 20 is a schematic view (1) of the embodiment of the drug injection system of the present invention, showing the needle output mechanism performing a needle outputting movement;
Fig. 21 is a schematic view (2) of the embodiment of the drug injection system of the present invention, showing the needle output mechanism performing a needle outputting movement;
Fig. 22 is a schematic view (3) of the embodiment of the drug injection system of the present invention, showing the needle output mechanism performing a needle outputting movement;
Fig. 23 is a schematic view (1) of the embodiment of the drug injection system of the present invention, showing the driving mechanism driving the piston rod to transport medicinal liquid;
Fig. 24 is a schematic view (2) of the embodiment of the drug injection system of the present invention, showing the driving mechanism driving the piston rod to transport the medicinal liquid;
Fig. 25 is a schematic view (1) of the embodiment of the drug injection system of the present invention, showing a contact end cap of the needle output mechanism being pressed to unlock the needle output element;
Fig. 26 is a schematic view (2) of the embodiment of the drug injection system of the present invention, showing the contact end cap of the needle output mechanism being pressed to unlock the needle output element; and
Fig. 27 is a schematic view of the embodiment of the drug injection system of the present invention, showing the needle output element of the needle output mechanism being pressed.

Description of the Reference Numbers:
1: injection channel module; 2: drug storage module; 3: driving module;
10: needle output mechanism; 11: casing; 111: base; 1111: first engaging hole; 1112: second engaging hole; 112: outer casing; 113: contact end cap; 1131: unlocking pushing portion; S: movement stroke; 114: resilient element; 12: needle output operation assembly; 121: needle output element; L: operation stroke; 1211: elongated rail groove; 1212: toothed driving portion; 12121: forward rotating rack segment; 12122: reverse rotating rack segment; 1213: engaging hook; 122: rotating element; 1220: threaded hole; 1221: gear portion; 123: needle seat plug; 1231: accommodating room; 1232: liquid injection room; 1233: connecting port; 1234: sealed portion; 1235: stop portion; 124: needle seat; 1241: threaded portion; 13: piercing assembly; 131: piercing needle; 132: conduit element; 1321: needle end mounting portion;
20: adapting mechanism; 21: adapting seat; 211: medicinal liquid inflow channel; 212: medicinal liquid outflow channel; 22: flow channel switching valve; 221: one-way input valve portion; 222: one-way output valve portion; 23: piston barrel; 230: piston chamber; 231: connecting end; 2311: medicinal liquid inlet; 2312: medicinal liquid outlet; 232: barrel opening end; 24: piston rod;
30: medicinal liquid storage container; 31: container body; 32: container connector; 321: connector body; 3210: infusion channel; 3211: container connecting end; 3212: infusion connecting end; 3213: infusion end portion; 32131: infusion branch channel; 3214: infusion plug; 32141: plug end;
40: outer covering;
50: housing; 51: housing body; 52: housing cover; 53: power supply unit; 54: electrical connector; 55: insulating sheet; 56: abutting sheet;
60: control unit; 61: switch; 62: activating switch; 63: confirming switch; 64: counting element; 65: indication light; 66: sound generator;
70: motor;
80: transmission assembly; 81: driving wheel; 82: connecting rod; 83: drive element.

### Description of the Present Invention

With reference to the drawings and embodiments of the present invention, the technical means adopted by the present invention to reach the intended purpose of the invention will be further described below.

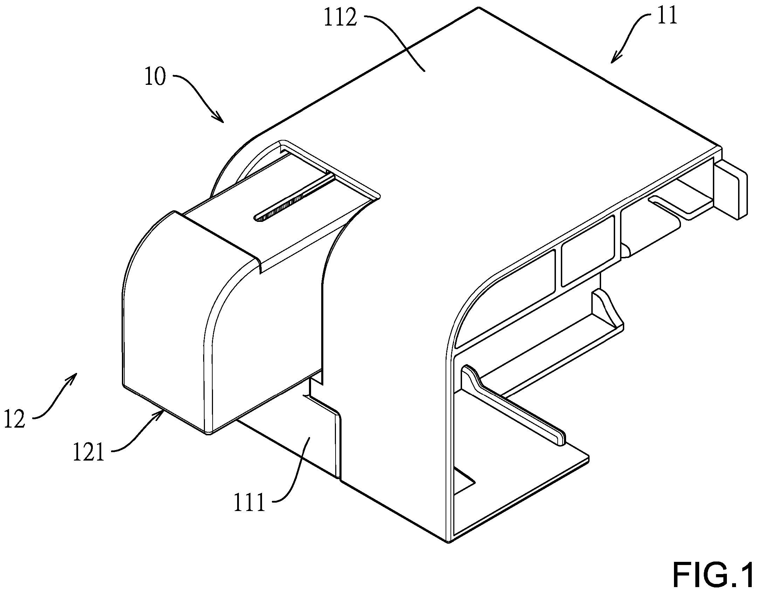

According to the description of the previously disclosed content of the invention, the present invention comprises a needle output mechanism, an injection channel module, and a drug injection system. As shown in Figs. 1, 6, and 11, the injection channel module 1 includes the needle output mechanism 10 and an adapting mechanism 20. The drug injection system includes the injection channel module 1, a drug storage module 2, and a driving module 3. Specific structures of the needle output mechanism 10, the injection channel module 1, and the drug injection system of the present invention are described in conjunction with the drawings as follows.

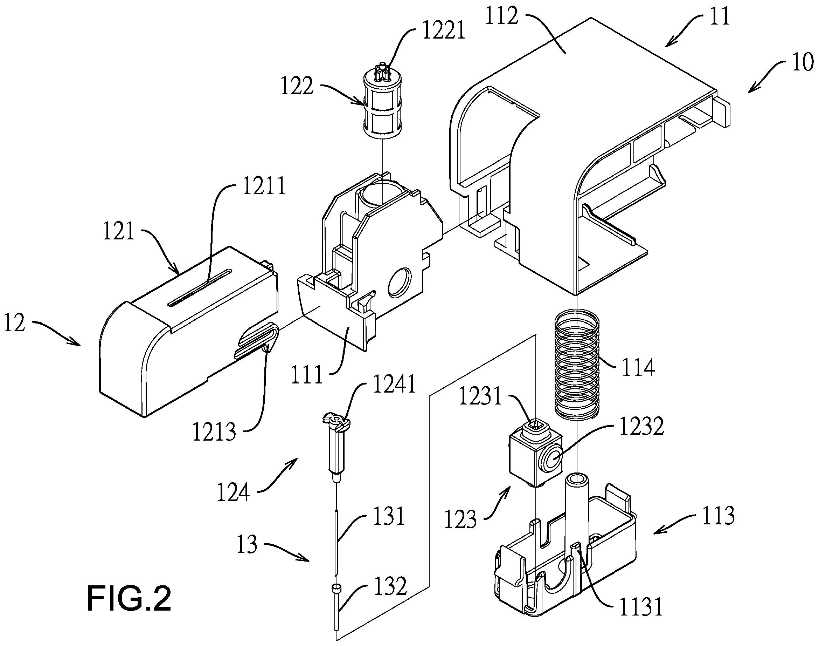

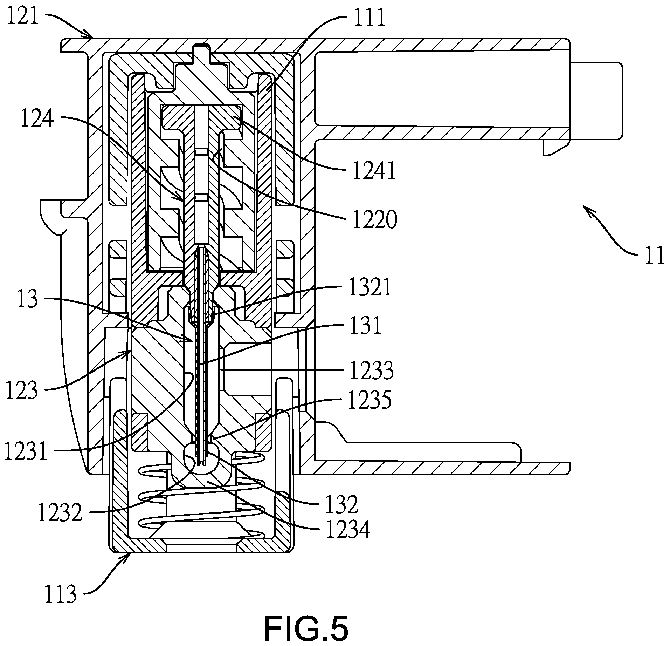

As shown in Figs. 1 to 5, an embodiment of the needle output mechanism 10 of the present invention is disclosed. As can be seen from the drawings, the needle output mechanism 10 includes a casing 11, a needle output operation assembly 12, and a piercing assembly 13. As shown in Figs. 1 to 5, the casing 11 has an assembling room. The needle output operation assembly 12 and the piercing assembly 13 are assembled in the assembling room of the casing 11. In the present embodiment, the casing 11 includes a base 111 and an outer casing 112 sleeved around the base 111. A back side of the outer casing 111 is mounted with a contact end cap 113 that is able to travel within a movement stroke S and a resilient element 114 that is disposed between the outer casing 112 and the contact end cap 113. Coil springs, flat springs or other resilient elements may be used as the resilient element. In the present embodiment, a compression spring is used as the resilient element 114.

As shown in Figs. 1 to 5, the needle output operation assembly 12 is mounted in the casing 11 and is connected with the piercing assembly 13. The needle output operation assembly 12 includes a needle output element 121, a rotating element 122, and a needle seat 124. The needle output element 121 is movably mounted in the casing 11. The rotating element 122 is rotatably mounted in the casing 11 and the rotating element 112 is able to be driven by the needle output element 121 to rotate. The needle seat 124 is movably mounted in the rotating element 122. The needle seat 124 is able to be driven by the rotating element 122 to move be between a first position and a second position.

As shown in Figs. 1 to 5, in the present embodiment, the needle output element 121 is mounted in the casing 11 to move linearly. The rotating element 122 is mounted in the base 111 of the casing 11 and is able to be driven by the needle output element 121 to rotate forwardly or reversely. In the present embodiment, the needle output element 121 is formed as a button-typed component and the rotating element 122 is formed as a sleeve-typed component. However, shapes of the needle output element 121 and the rotating element 122 are not limited thereto.

As shown in Figs. 1 to 5, a gear portion 1221 is provided on an upper end of the rotating element 122 and a toothed driving portion 1212 is provided in the needle output element 121. The toothed driving portion 1212 may connect with or disconnect from the gear portion 1221. When the needle output element 121 is pushed, through the transmission between the toothed driving portion 1212 and the gear portion 1221 that are meshed, the rotating element 122 is driven to rotate in a forward direction and then in a reverse direction.

As shown in Figs. 1 to 5, in the present embodiment, the needle output element 121 has an elongated rail groove 1211 and a length of the elongated rail groove 1221 is defined as an operation stroke L. The toothed driving portion 1212 includes a forward rotating rack segment 12121 and a reverse rotating rack segment 12122. The forward rotating rack segment 12121 and the reverse rotating rack segment 12122 are two different sections respectively disposed along a Z-axis of the elongated rail groove 1211 and are disposed on two opposite sides. A length of the forward rotating rack segment 12121 and a length of the reverse rotating rack segment 12122 are half of the operation stroke L. The gear portion 1221 of the rotating element 122 is disposed in the elongated rail groove 1211, and with movement of the needle output element 121 along the operation stroke L, the gear portion 1221 of the rotating element 122 is able to mesh with either the forward rotating rack segment 12121 or the reverse rotating rack segment 12122 respectively, such that the rotating element 122 is able to be driven to rotate in the forward direction and in the reverse direction.

As shown in Figs. 1 to 5, the needle output operation assembly 12 may further include a needle seat plug 123. The needle seat plug 123 is a soft component made of rubber material or the same. The needle seat plug 123 is mounted in the base 111 and adjacent to a back surface of the casing 11. The needle seat plug 123 has an accommodating room 1231, a liquid injection room 1232 disposed at a lower end of the accommodating room 1231, and a connecting port 1233 disposed on a side of the liquid injection room 1232 and communicating with the liquid injection room 1232, and a sealed portion 1234 disposed at a lower end of the liquid injection room 1232 and exposed on the back surface of the casing 11. A stop portion 1235 is provided between the accommodating room 1231 and the liquid injection room 1232. The needle seat 124 is movably mounted in the rotating element 122. The needle seat 124 is able to be driven by the rotating element 122 to move upwardly and downwardly between the rotating element 122 and the needle seat plug 123.

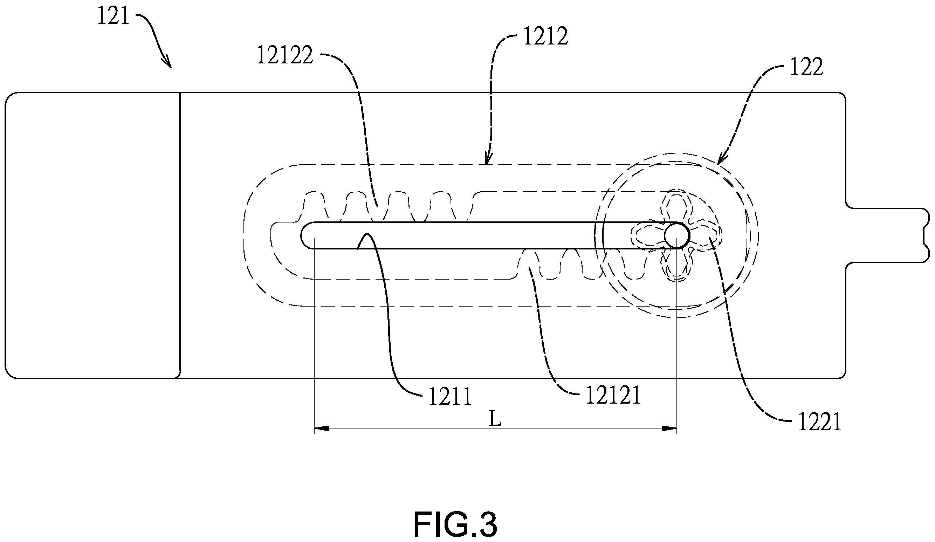

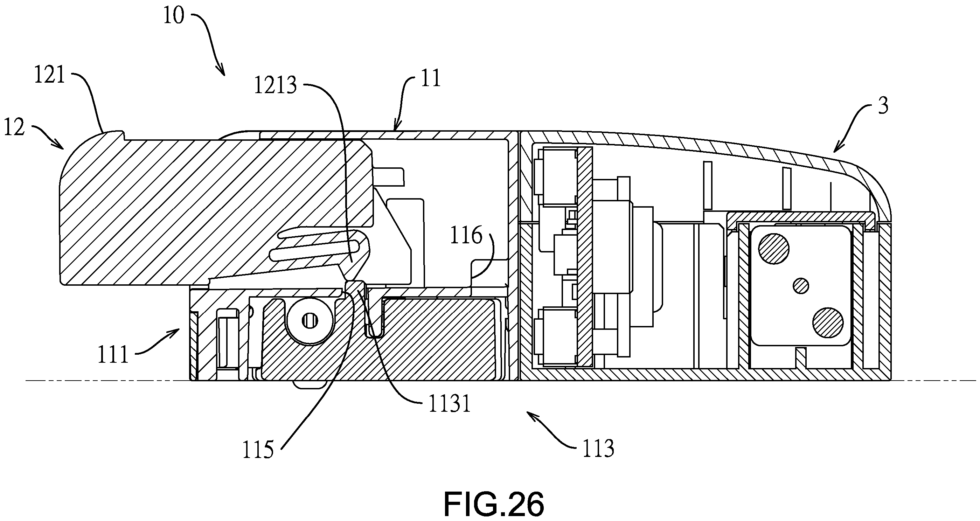

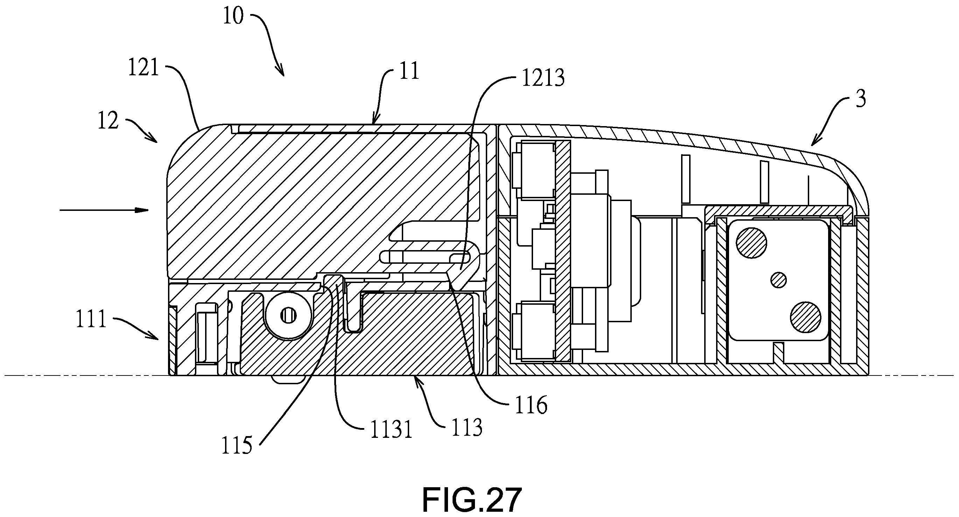

As shown in Figs. 3 and 25 to 27, the needle output element 121 is mounted in the base 111 and is able to move within a distance of the operation stroke L. The needle output element 12 is able to be locked in the base 111 at a starting position and an end position of the operation stroke L respectively to be in a locked state. Resilient force of the resilient element 114 acts on the contact end cap 113, causing part of the contact end cap 113 to protrude from a back side of the outer casing 112. The contact end cap 113 is able to be forced to enter the outer 112 to release the needle output element 12 that is located at the starting position of the operation stroke L from the locked state.

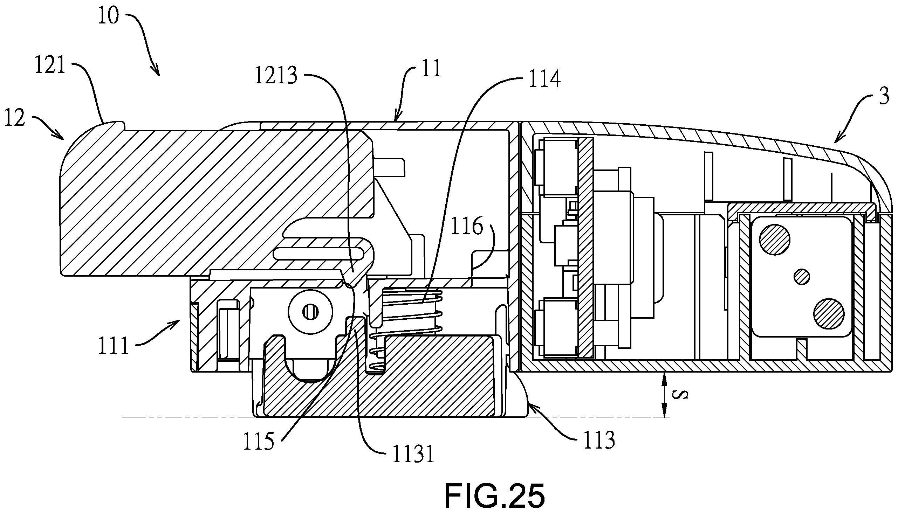

As shown in Figs. 25 to 27, in the present embodiment, the base 111 has a first engaging hole 1111 disposed on the starting position of the operation stroke L and a second engaging hole 1112 disposed on the end position of the operation stroke L. An engaging hook 1213 is provided on a rear end of the needle output element 121. The engaging hook 1213 is able to selectively engage in the first engaging hole 1111 or the second engaging hole 1113. The contact end cap 113 has an unlocking pushing portion 1131. When the contact end cap 113 is forced to enter the outer casing 112, the unlocking pushing portion 1131 is able to push the engaging hook 1213 of the needle output element 121 to disengage from the first engaging hole 1112 to release the needle output element 121 that is locked from the locked state.

As shown in Figs. 1 to 5, the piercing assembly 13 is mounted in the casing 11 and the piercing assembly 13 is able to be operated to a needle output state and a needle retraction state. The piercing assembly 13 includes a piercing needle 131 and a conduit element 132. The piercing needle 131 is connected with the needle seat 124 of the needle output operation assembly 12. The conduit element 132 is able to be combined with the piercing needle 131. In the needle output state, the piercing assembly 13 that is formed by combining the conduit element 132 and the piercing needle 131 is driven by the needle output operation assembly 12 to cause at least part of the piercing assembly 13 to protrude out of the casing 11. In the needle retraction state, the piercing needle 131 moves upward along with the needle seat 124 to retract the piercing needle 131.

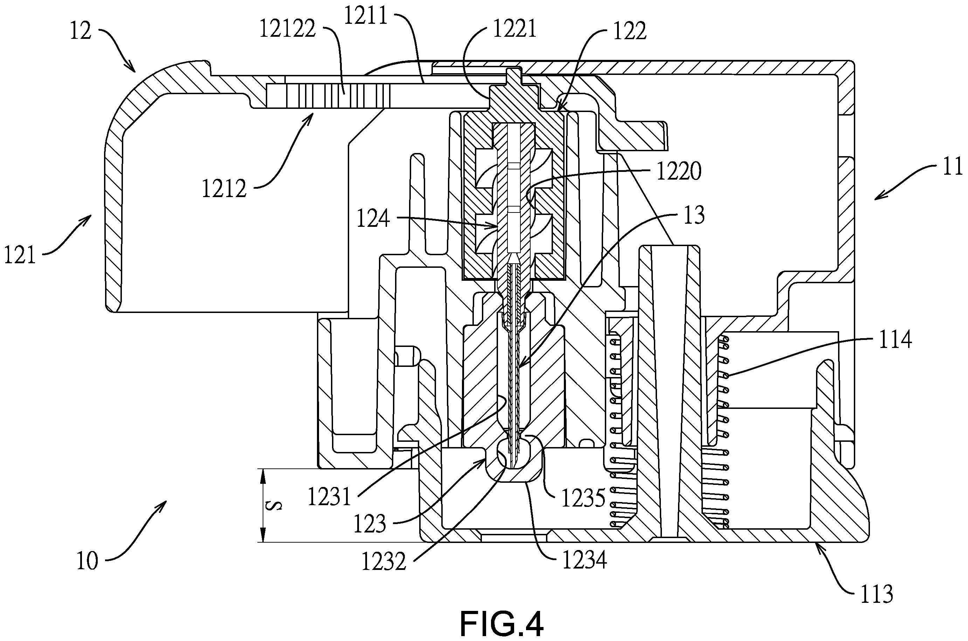

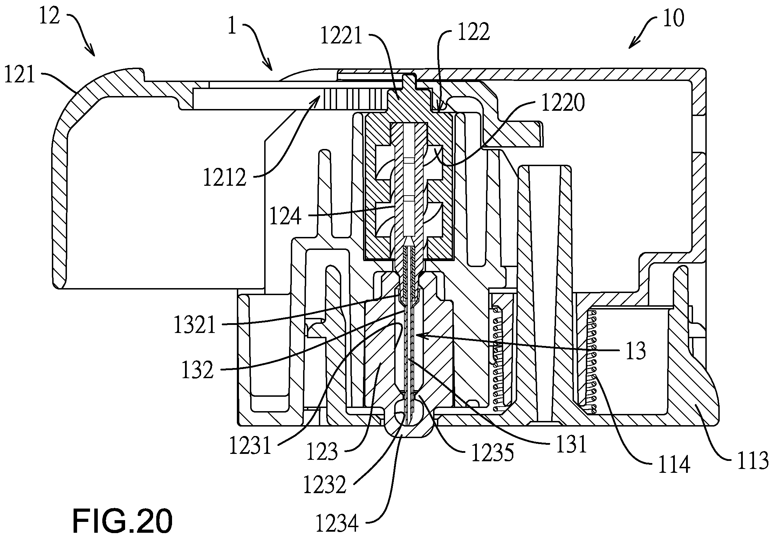

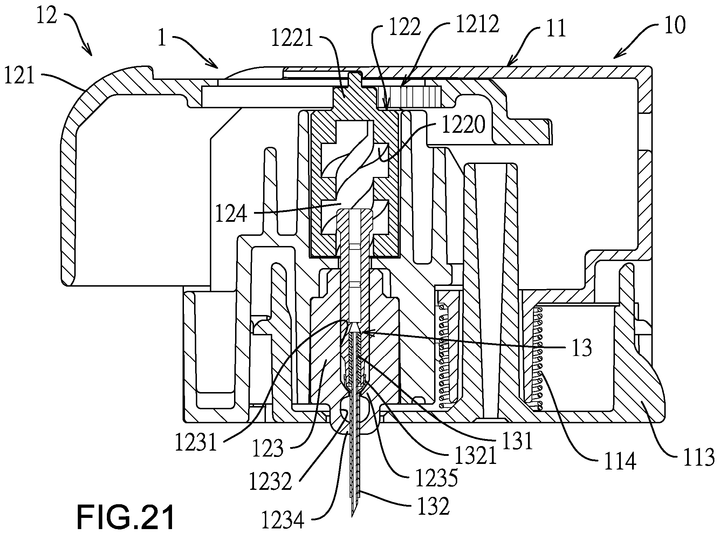

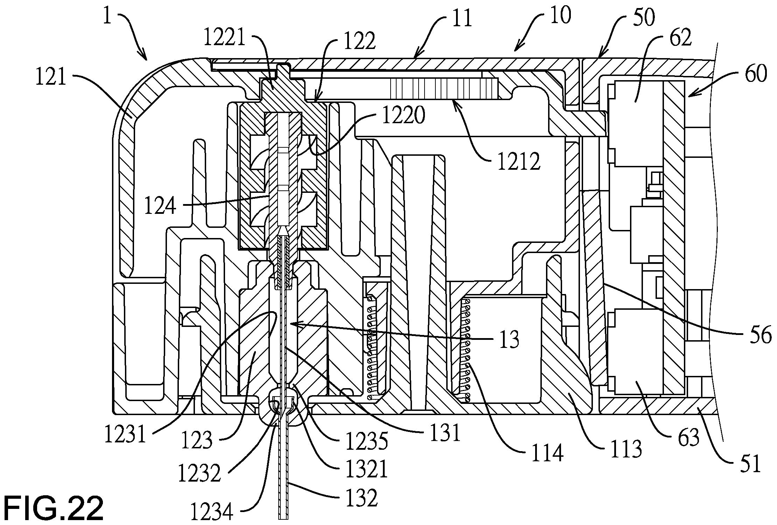

As shown in Figs. 4, 5 and 20 to 22, in the present embodiment, an upper section of the piercing needle 131 of the piercing assembly 13 is connected with the needle seat 124 and a lower section of the piercing needle 131 protrudes downward into the needle seat plug 123. The conduit element 132 is disposed inside the needle seat plug 123 and is sleeved around the lower section of the piercing needle 131. A needle end mounting portion 1321 is provided on an upper end of the conduit element 132 and the needle end mounting portion 1321 is sleeved onto a lower end of the needle seat 124. In the needle output state, the piercing assembly 13 that is formed by combining the conduit element 132 and the piercing needle 131 is operable to pierce downward through the sealed portion 1234 on a bottom of the needle seat plug 123. In the needle retraction state, the piercing needle 131 moves upward along with the needle seat 124 to retract the piercing needle 131 and the conduit element 132 is detained. Part of the conduit element 132 remains protruding out of the sealed portion 1234 and being disposed outside the casing 11.

As shown in Figs. 1 to 5, in the present embodiment, the rotating element 122 and the needle seat 124 are connected through threads. That is, the rotating element 122 has a threaded hole 1220 formed therein, and a threaded portion 1241 is provided on an outer side surface of the needle seat 124 and engages with the threaded hole 1220 of the rotating element 122, such that forward rotation and reverse rotation of the rotating element 122 is able to drive the needle seat 124 to move up and down. In the present embodiment, to further set the needle output element 121 in the operation stroke L, the needle output element 121 is able to firstly drive the rotating element 122 to rotate forwardly to drive the needle seat 124 to push the piercing assembly 13 to move downward to output the needle. Then the needle output element 121 drives the rotating element 122 to rotate reversely to drive the needle seat 124 and the piercing needle 131 that is connected therewith to move upward to retract the needle, allowing part of the conduit element 132 to remain protruding out of the casing 11.

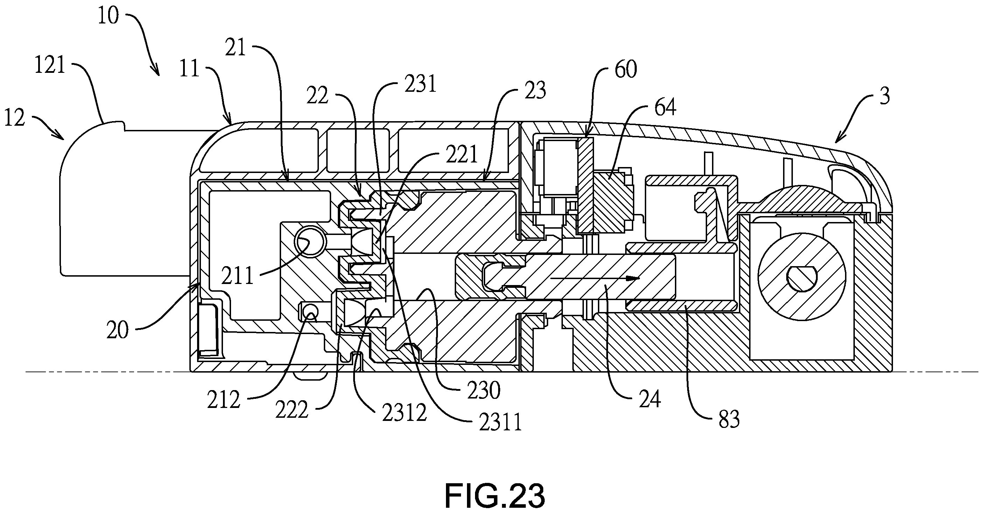

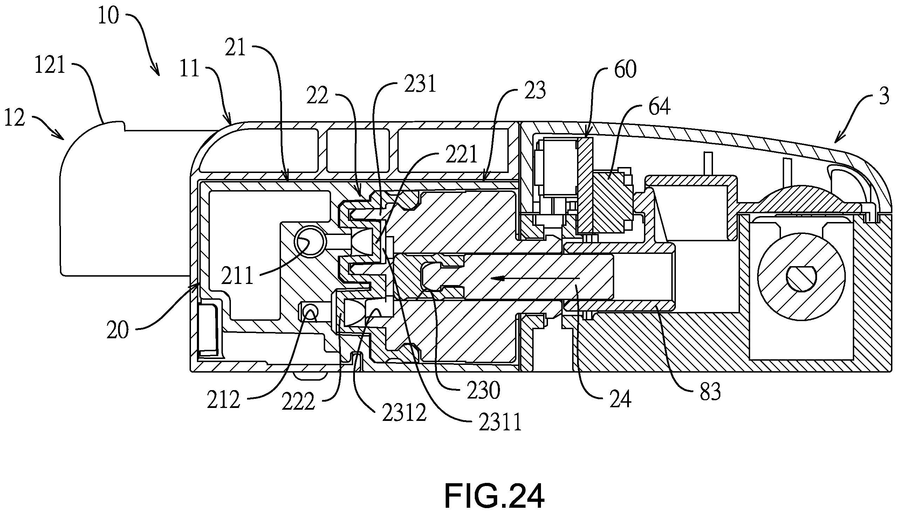

In the present embodiment, the needle output mechanism10 may be used in a syringe. With reference to Figs. 3 to 5 and 20 to 27, when the needle output mechanism10 has not been used, the piercing assembly 13 of the needle output mechanism10 is hidden in the casing 11. When outputting the needle, a user makes a back side of the needle output mechanism 10 face a site to be injected on the human body and exerts pressuring force on the casing 11 to make the contact end cap 113 on a back side of the casing 11 to be retracted in the casing 11 due to pressure and the unlocking pushing portion 1132 of the contact end cap 113 push the engaging hook 1213 of the needle output element 121 away from the first engaging hole 1112 to release the needle output element 121 that is located at the starting position of the operation stroke L from the locked state until the back side of the casing 11 is attached to the site to be injected on the human body.

Then with moving the needle output element 121 from the starting position to the end position of the operation stroke L by pressing the needle output element 121, the needle output element 121 drives the rotating element 122 to rotate in the forward direction firstly and drives the needle seat 124 and the piercing assembly 13 that is connected therewith and is formed by combining the conduit element 132 and the piercing needle 131 to be pushed out from the needle seat plug 123 and pierce into the human body. As the needle output element 121 further moves to the end position of the operation stroke L, the rotating element 122 is further driven to rotate in the reverse direction, the needle seat 124 and the piercing needle 131 that is connected therewith are retracted into the needle seat plug 123 while the conduit element 132 is detained. Part of the conduit element 132 remains protruding out of the needle seat plug 123 and being disposed outside the casing 11. The conduit element 32 stays at a position of piercing into the human body. Meanwhile, the engaging hook 1213 of the needle output element 121 that has been moved to the end position of the operation stroke L is able to engage in the second engaging hole 111 of the base 12 to keep the needle output element 121 in a locked state and allow the user to inject medicinal liquid without pressing the needle output element 121. After the injection of the medicinal liquid is complete and the needle output mechanism 10 is removed from the human body, the contact end cap 113 is pushed back by the resilient force of the resilient element 114, returns to its original position and is mounted around and outside the lower section of the piercing needle 131 to provide safety protection.

The conduit element 132 may be a soft needle or a soft tube such as a heat shrinkable soft tube. However, the conduit element 132 is not limited thereto. As long as an element is suitable to be combined with the piercing needle 131 and pierced into the human body, is suitable to be left in the human body and forms a channel that allows liquid to flow into the human body through the channel, said element is suitable for use as the conduit element 132.

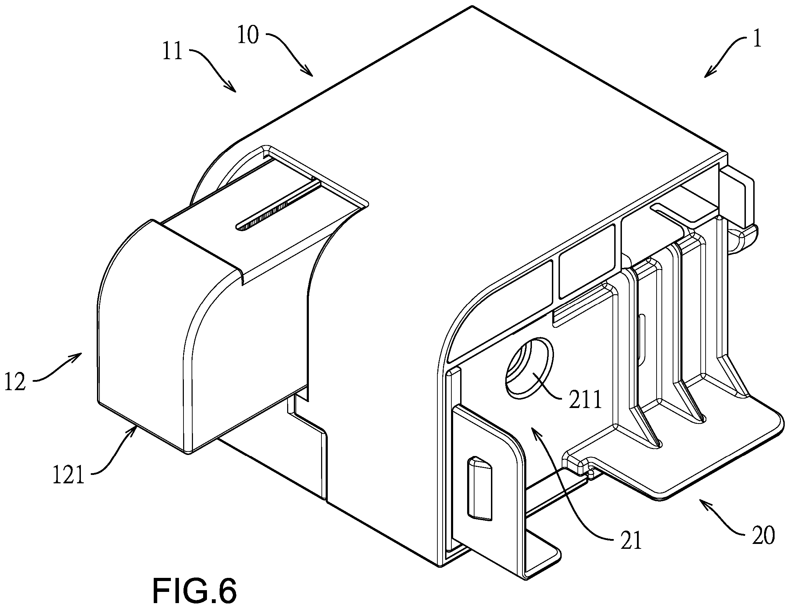

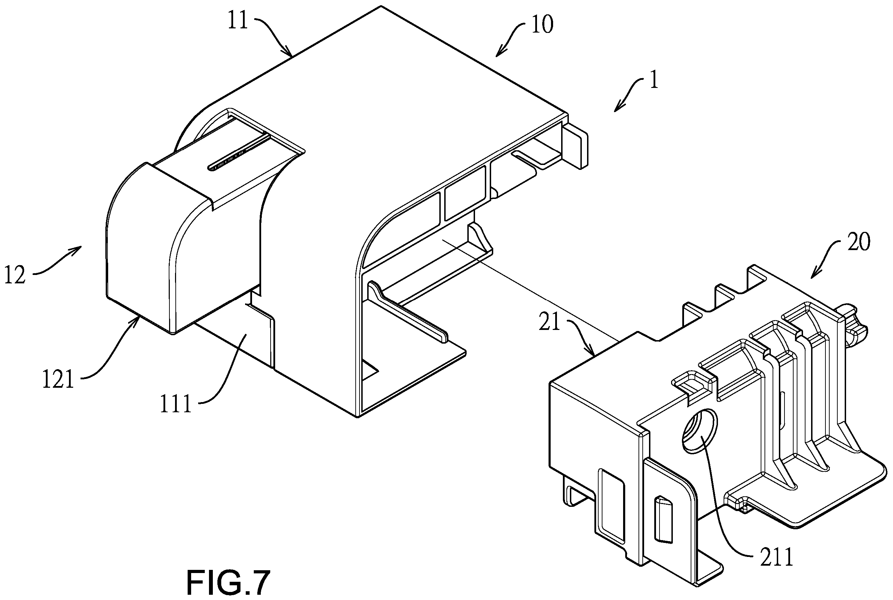

As shown in Figs. 6 and 7, an embodiment of the injection channel module 1 of the present invention is disclosed. As can be seen from the drawings, the injection channel module 1 includes a needle output mechanism 10 and an adapting mechanism 20. The structure of the needle output mechanism 10 is as disclosed above and will be not repeated below.

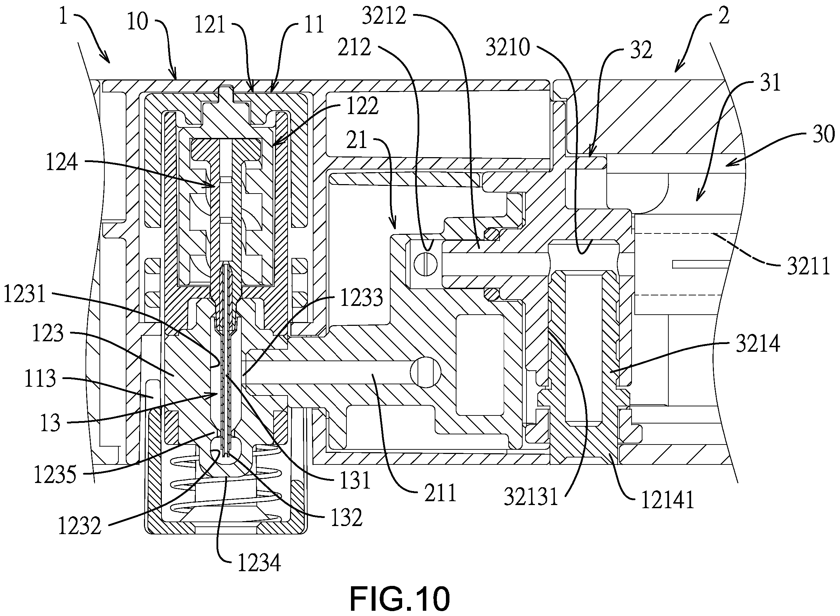

As shown in Figs. 7, 8 and 10, the adapting mechanism 20 includes an adapting seat 21, a flow channel switching valve 22, a piston barrel 23, and a piston rod 24. The adapting seat 21 is able to be assembled to a side of the casing 11. The adapting seat 21 has a medicinal liquid inflow channel 211 and a medicinal liquid outflow channel 212. The flow channel switching valve 22 is mounted in the adapting seat 21, and the flow channel switching valve 22 includes a one-way input valve portion 221 and a one-way output valve portion 222. Two ends of the piston barrel 23 are respectively a connecting end 231 and a barrel opening end 232. The connecting end 231 is disposed in the adapting seat 21 and is connected to the flow channel switching valve 22. The barrel opening end 232 is able to protrude out of the adapting seat 21. The piston barrel 23 has a piston chamber 230 formed therein. The connecting end 231 has a medicinal liquid inlet 2311 and a medicinal liquid outlet 2312 communicating with the piston chamber 230. The medicinal liquid inlet 2311 is connected to the one-way input valve portion 221. The medicinal liquid outlet 2312 is connected to the one-way output valve portion 222. The piston rod 24 is assembled in the piston chamber 230 of the piston barrel 23 and protrudes out of the adapting seat 21. The piston rod 24 is able to be driven to move linearly in the piston chamber 230 of the piston barrel 23. When the piston rod 24 moves backward, the medicinal liquid is drawn into the piston chamber 230 through the medicinal liquid inflow channel 211 and the one-way input valve portion 221 in one way. Then when the piston rod 24 moves forward, the medicinal liquid in the piston chamber 230 is output to the medicinal liquid outflow channel 212 through the one-way output valve portion 222 in one way to input the medicinal liquid for injection into the injection mechanism 10.

As shown in Figs. 7, 8 and 10, the piston rod 24 of the injection channel module 1 may be operated manually, or with a manual driving module 3 or an electric driving module 3 that is externally connected to the piston rod 24, to drive the injection channel module 1 to inject the medicinal liquid. In the injection channel module 1, the injection action of introducing the medicinal liquid into the injection mechanism 10 through the switching mechanism 20 is as described above and will not be repeated below.

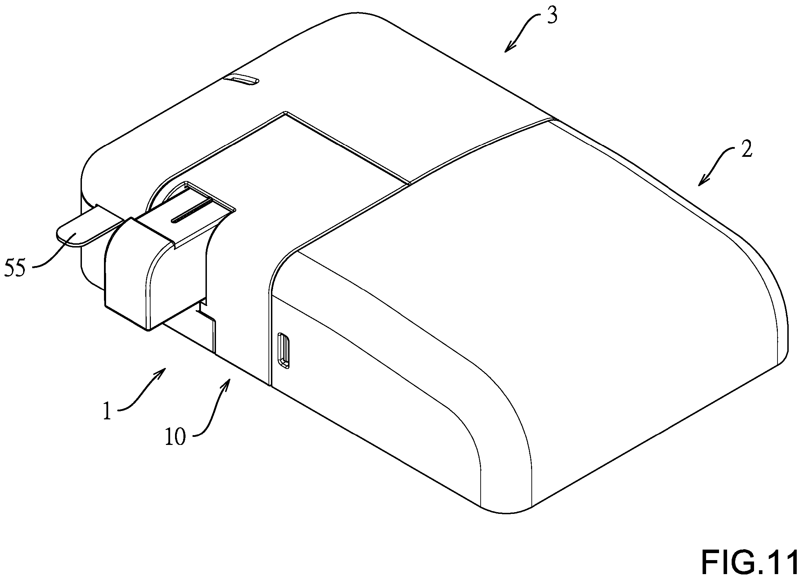

As shown in Figs.11 to 12, an embodiment of the drug injection system of the present invention is disclosed. The drug injection system includes said injection channel module 1, a drug storage module 2, and a driving module 3. The structure of the injection channel module 1 is as disclosed above and will be not repeated below.

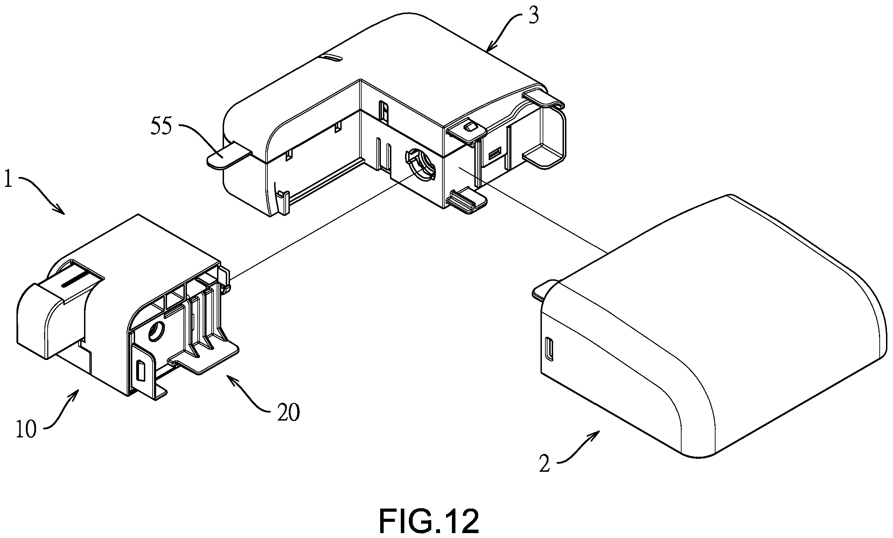

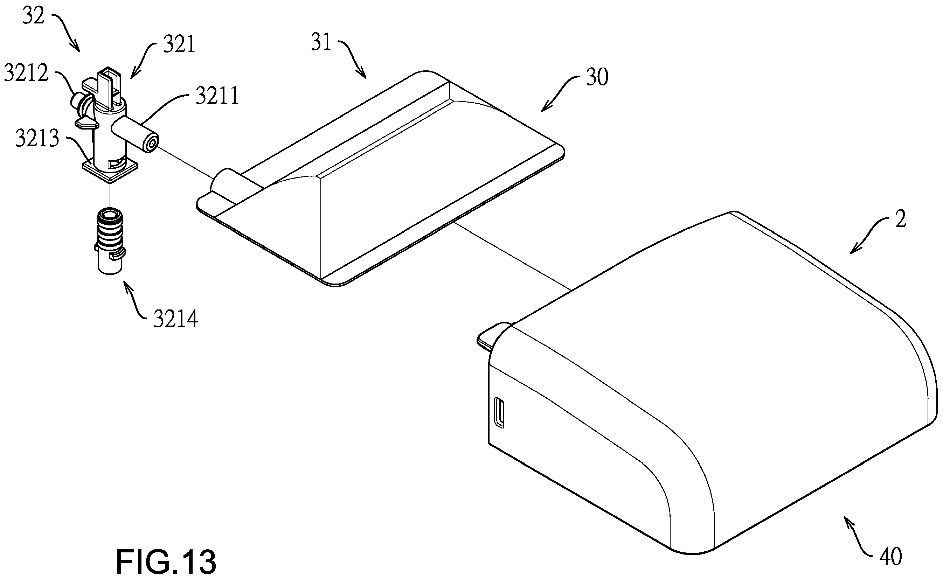

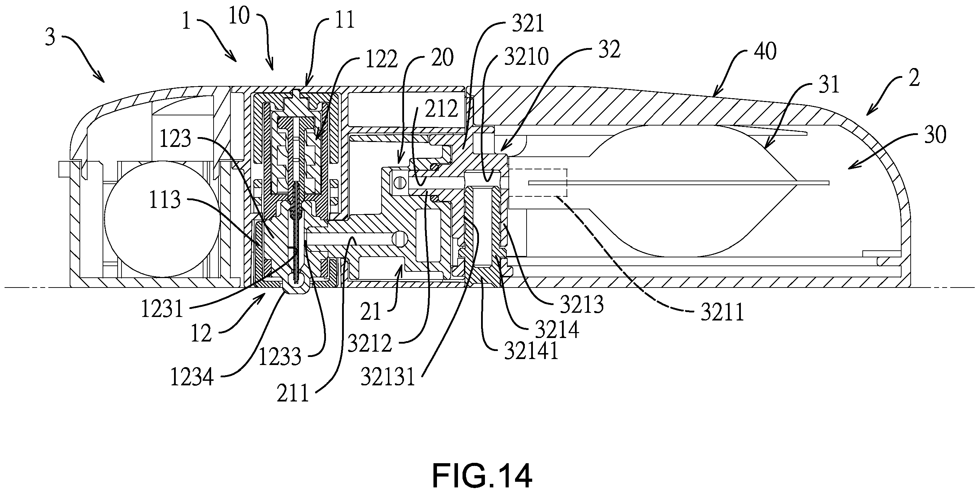

As shown in Figs. 12 to 14, the drug storage module 2 includes a medicinal liquid storage container 30 and an outer covering 40. The medicinal liquid storage container 30 includes a container body 31 and a container connector 32. The container body 31 is able to be filled with the medicinal liquid inside. That is, the container body 31 is a component that is able to be pre-filled with the medicinal liquid. Or the container body 31 is a component that is not originally filled with the medicinal liquid and the predetermined medicinal liquid is injected into the container body 31 before use. The container connector 32 is assembled to the container body 31. The medicinal liquid storage container 30 is able to be connected to the medicinal liquid inflow channel 211 of the adapting mechanism 20 of the injection channel module 1 with the container connector 32. The outer covering 40 is able to be sleeved around the medicinal liquid storage container 30 and connected with the casing 11 of the needle output module.

As shown in Figs. 12 to 14, in the present embodiment, a bag-shaped component, a bottle-shaped component or the same for storing the medicinal liquid is able to be used as the container body 31. In the present embodiment, a bag-shaped component is used as the container body 31, but it is not limited thereto. The container connector 32 is connected to the container body 31. The container connector 32 has an infusion channel 3210 that communicates with the container body 31 and is connected to the medicinal liquid inflow channel 211 of the adapting mechanism 20 through the infusion channel 3210.

As shown in Figs. 12 to 14, the container connector 32 includes a connector body 321. The connector body 321 includes a container connecting end 3211 and an infusion connecting end 3212. The infusion channel 3210 extends from the container connecting end 3211 to the infusion connecting end 3212, is connected with the container body 31 through the container connecting end 3211, and is connected with the medicinal liquid inflow channel 211 of the adapting mechanism 20 through the infusion connecting end 3212.

In the present embodiment as shown in Figs. 12 to 14, the connector body 321 may further includes an infusion end portion 3213. The infusion end portion 3212 has an infusion branch channel 32131 communicating with the infusion channel 3210 and an infusion plug 3214 mounted in the infusion branch channel 32131, and seals the infusion branch channel 32131 with the infusion plug 3214. The infusion plug 3214 has a plug end 32141 exposed outside the connector body 321. The plug end 32141 is exposed on a back surface of the outer covering 40. Thus, by puncturing the plug end 32141 with a syringe, the medicinal liquid to be added can flow through the infusion branch channel 32131 and the infusion channel 3210 to enter the container body 31 of the medicinal liquid storage container 30.

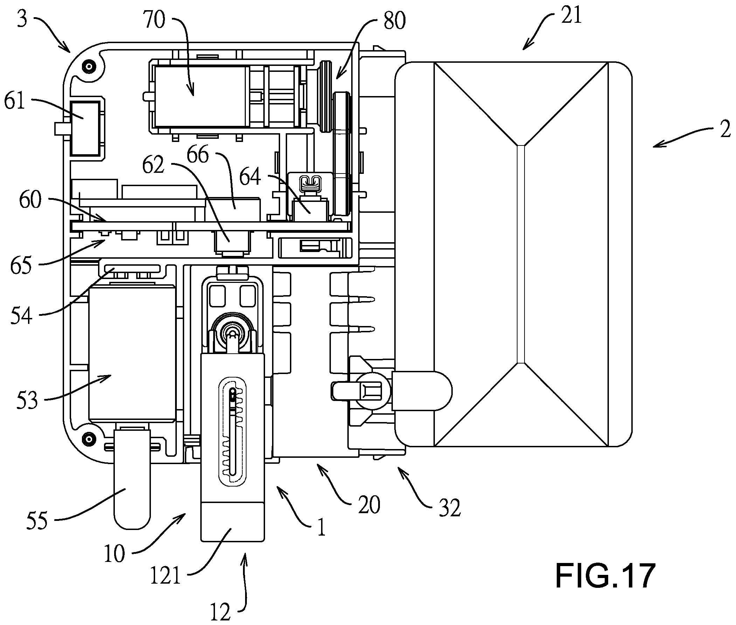

As shown in Figs. 12 and 15 to 17, the driving module 3 is an electric driving module 3. The driving module 3 is detachably assembled to the injection channel module 1. The driving module 3 includes a power supply unit 53 or is able to be connected to an external power supply. The driving module 3 includes a housing 50, a control unit 60, a motor 70, and a transmission assembly 80. The control unit 60, the motor 70 and the transmission assembly 80 are all mounted in the housing 50. The motor 70 is electrically connected to and is controlled by the control unit 60. The control unit 60 has a switch 61 exposed outside the housing 50. The transmission assembly 80 is connected to the motor 70 and is detachably connected to the piston rod 24 of the adapting mechanism 20. The control unit 60 determines the operation timing of the motor 70 according to a preset injection mode, so that the motor 70 can be started under control and drive the piston rod 24 to work through the transmission assembly 80.

As shown in Figs. 12 and 15 to 17, in the present embodiment, the housing 50 includes a housing body 51 and a housing cover 52 mounted on the housing body 51. A power supply unit 53 is able to be mounted in the housing 50. Said power supply unit 53 may be a battery, a battery pack composed of multiple batteries, or the like. Two electrical connectors 54 that electrically connected to the control unit 60 are mounted in the housing 50. Two electrode terminals of said power supply unit 53 are respectively connected to the two electrical connectors 54, and are electrically connected to the control unit 60 through the two electrical connectors 54, and provide electrical power to the control unit 60 of the driving module 3 and the motor 70. Before the driving module 3 is not used, an insulating sheet 53 is provided between an end of the power supply unit 53 and one of the electrical connectors 54 that corresponds in position thereto for insulation and isolation. The insulating sheet 53 protrudes out of the housing 50. When using, the insulating sheet 53 is pulled out to allow the power supply unit 53 to be electrically connected with the electrical connectors 54.

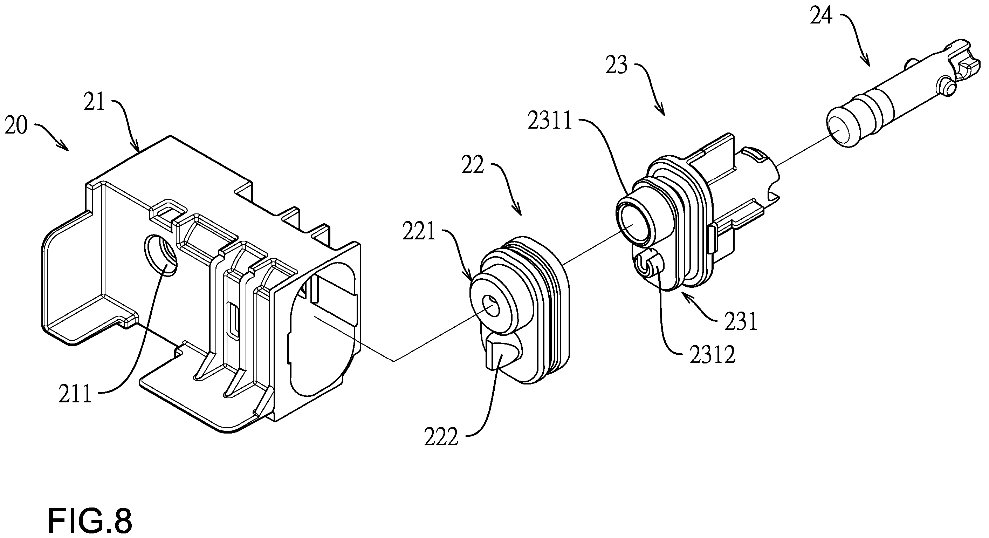

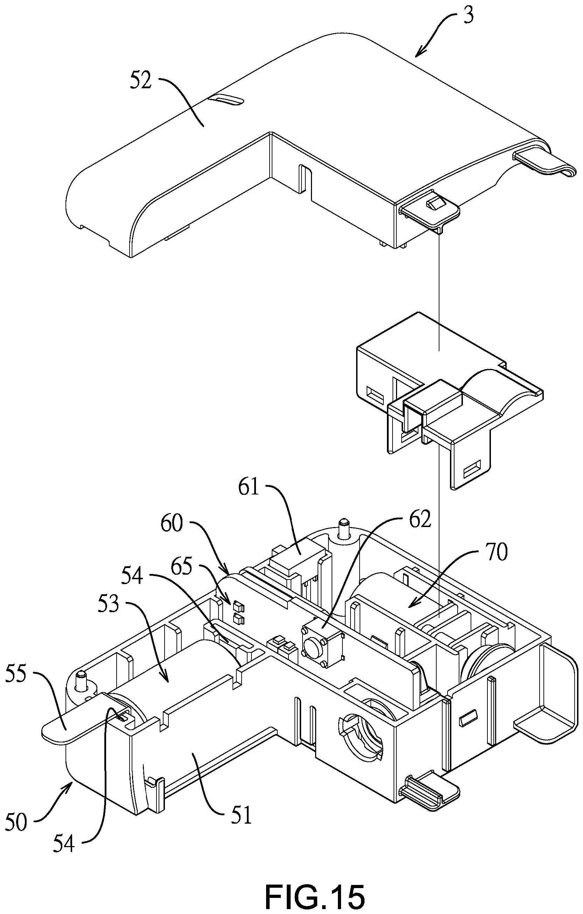

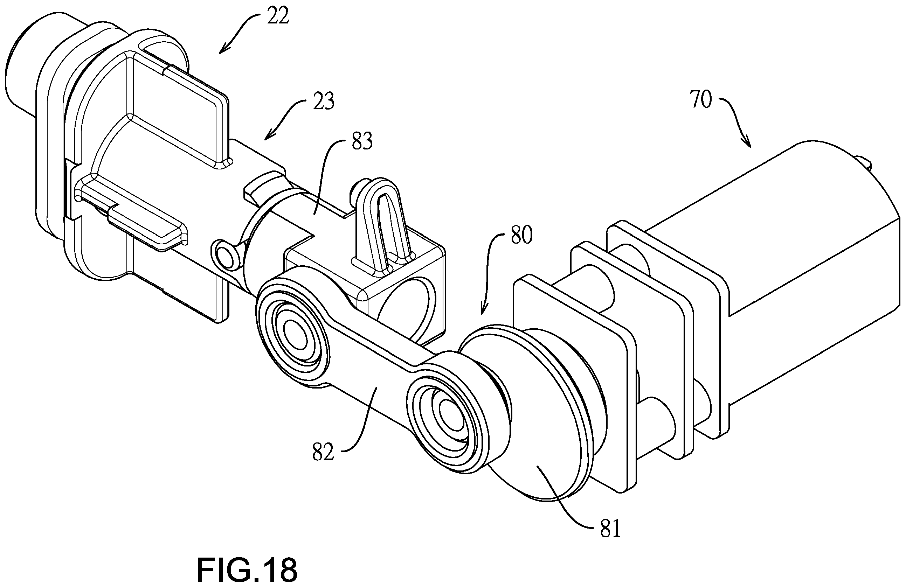

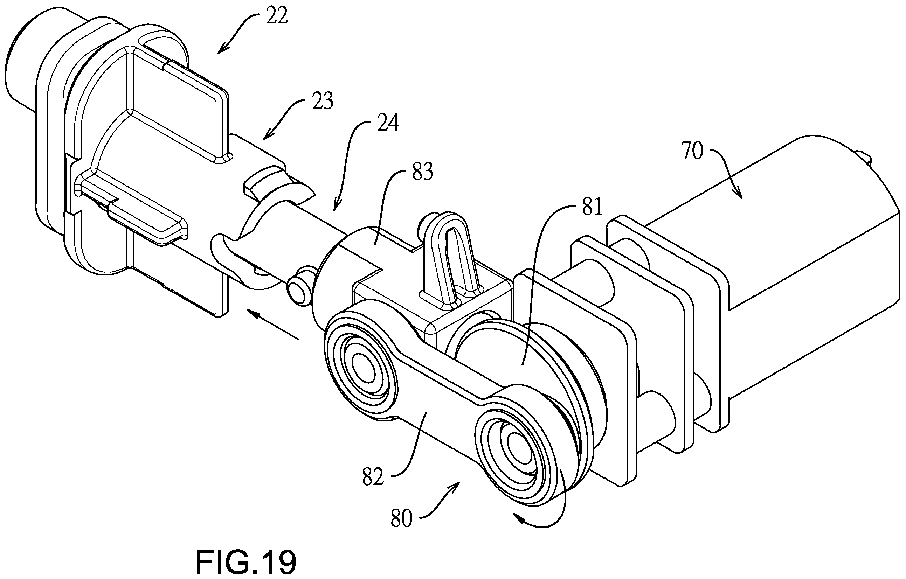

As shown in Figs. 15 to 17 and 18 to 19, in the present embodiment, the transmission assembly 80 includes a driving wheel 81, a connecting rod 82 and a drive element 83. The driving wheel 81 is assembled to a central shaft of the motor 70. An end of the connecting rod 82 is eccentrically connected pivotally to the driving wheel 81. Another end of the connecting rod 82 is connected to the drive element 83 and is detachably connected to a distal end of the piston rod 24 through the drive element 83. Preferably, a movement direction of the connecting rod 82 and the drive element 83 is parallel with a central axis of the piston rod 24, so that the transmission assembly 80 drives the piston rod 24 to linearly reciprocate smoothly in the piston barrel 23. With reference to Figs. 8, 18 to 19, a connection structure between the drive element 83 and the distal end of the piston rod 24 may be a middle L-shaped connecting groove formed on the piston rod 24, and a middle hole and a connecting protrusion, which is disposed on an inner sidewall of the middle hole, of the drive element 83. Thus, the piston rod 24 is axially mounted in the middle hole of the drive element 83, and the connecting protrusion straightly moves along the L-shaped connecting protrusion and is rotated to engage in position in a distal end of the connecting groove, such that assembly of the piston rod 24 and the drive element 83 is facilitated.

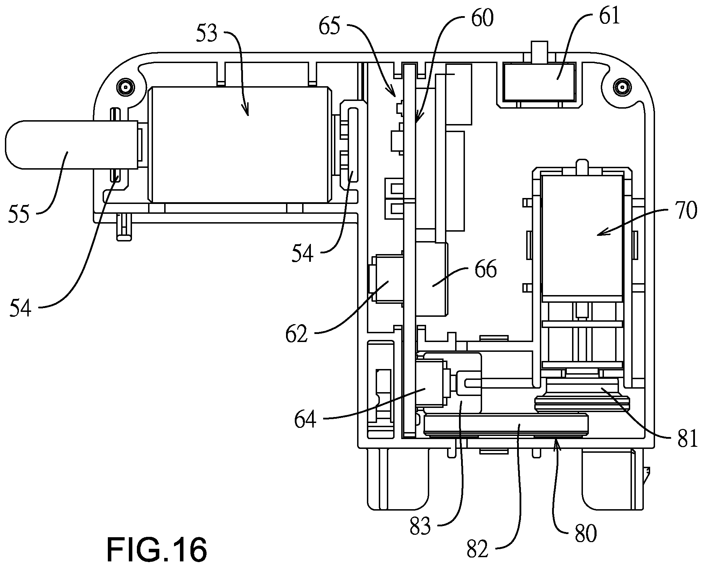

As shown in Figs. 16 to 17 and 20 to 22, the driving module 3 further includes an activating switch 62. The activating switch 62 is mounted on the housing 50 and is disposed at the end position of the moving stroke L of the needle output element 121. The activating switch 62 is electrically connected to the control unit 60. When the needle output element 121 is pressed to the end position, the activating switch 62 is activated, so that the control unit 60 drives the motor 70 to infuse liquid.

As shown in Figs. 16 to 17 and 20 to 22, the driving module 3 further includes a confirming switch 63. The confirming switch 63 is electrically connected to the control unit 60, and the confirming switch 63 is disposed at an end position of the movement stroke S of the contact end cap 113. Or an abutting sheet 56 is further provided on the housing body 50 and corresponds in position to the confirming switch 63. When the contact end cap 113 is indeed pushed inside the casing 11, the confirming switch 63 is activated directly or via the abutting sheet, so as to confirm that it has been installed on the human body and is in a state of preparation for injection (i.e. standby mode). If the contact end cap 113 has not been pushed inside the casing 11 to activate the confirming switch 63, it is on an idle state.

As shown in Figs. 16 to 17 and 18 to 19, the driving module 3 further includes a counting element 64. The counting element 64 is mounted in the housing 50 and is electrically connected to the control unit 60. The counting element 64 is disposed at a predetermined position of a movement path of the drive element 83. When the drive element 83 is moved to the predetermined position, the counting element 64 is activated and the counting element 64 outputs an injection signal to the control unit 60. The control unit 60 receives the injection signal and calculates a number of injections, so as to determine dose of injection.

As shown in Figs. 11 to 17, the driving module 3 further includes an indication light 65. The indication light 65 is electrically connected to the control unit 60 and can shine on the housing 50. The indication light 65 can be controlled to generate signals for indicating on, off and status. The indication light 65 includes a first light-emitting element and a second light-emitting element that emit light of different colors. For instance, the first light-emitting element is a blue light-emitting element capable of emitting blue light, and the second light-emitting element is a red light-emitting element capable of emitting red light. However, the first light-emitting element and the second light-emitting element that emit light of different colors are not limited to the blue light-emitting element and the red light-emitting element. Under the control of the control unit 60 according to different statuses, the first light-emitting element (the blue light-emitting element) and the second light-emitting element (the red light-emitting element) of the indication light 65 will be lighted up or turned off respectively. In addition, the driving module 3 may further include a sound generator 66. The sound generator 66 may be a buzzer and is electrically connected to the control unit 60, so that the sound generator 66 can be controlled to generate sounds for indicating on, off and status.

In the drug injection system of the present invention, an adhesive film and a release film disposed on an outer surface of the adhesive film are provided on the back surface of the casing 11 of the needle output mechanism 10, the back surface of the outer covering 40 of the drug storage module 2 and a back surface of the housing 50 of the driving module 3. When using the drug injection system, the release film is peeled first. After the release film has been peeled off, the adhesive film on a back surface of the drug injection system is able to be adhered to the human body for injection. Preferably, the adhesive film is air permeable.

As shown in Figs. 11 and 20 to 24, regarding to usage of the drug injection system of the present invention, the needle output mechanism 10 and the adapting mechanism 20 can pre-assembled into the injection channel module 1 and be connected to the drug storage module 2 and the driving module 3. In this way, the contact end cap 113 protrudes out of the casing 11 due to the resilient force of the resilient element 114 and the air permeable adhesive film with the release film is pre-attached to the back surface of the drug injection system. After being sterilized, said drug injection system can be pre-stored in a sterilized container which is sealed with a sterilized film paper.

When using, tear off the sterilized film paper on the container to take out the drug injection system. An appearance of the drug injection system is confirmed normal by observing whether the contact end cap 113 is protruding outside the casing 11 and checking whether the release film is damaged or contaminated. Confirm that the appearance of the drug injection system is normal before using it.

On the other hand, the user has to first confirm whether the medicinal liquid filled in the container body 31 of the drug storage module 2 of the drug injection system is correct. Make sure the drug is correct before injecting. If the container body 31 of the drug storage module 2 is an empty container that is not filled with medicinal liquid in advance, it is necessary to draw the correct medicinal liquid with a syringe before injection and then use a needle of the syringe to pierce into the infusion end portion 3213 of the container connector 32 to allow the medicinal liquid in the syringe to be injected into the container body 31 through the container connector 32, and then it is able to inject.

Moreover, as shown in Figs. 15 to 17, before injection, the insulating sheet 55 is removed, such that the power supply unit 53 of the driving module 3 electrically contacts the electrical connectors 54 to be electrically connected to the control unit 60. When the switch 61 is switched to a on state, the indication light 65 comes on. Afterwards, the release film on the back surface of the drug injection system is peeled, the back surface of the drug injection system faces the site to be injected on the human body, and the contact end cap 113 contacts the site to be injected on the human body and is pressed. When the contact end cap 113 is pressed, the resilient element 114 is compressed and is retracted into the casing 11, and the air permeable adhesive film on the back surface of the drug injection system is adhered to the human body for positioning. As shown in Figs. 25 to 27, as the contact end cap 113 is pressed to be retracted to the end position of the moving stroke S in the casing 11, the unlocking pushing portion 1131 of the contact end cap 113 pushes the engaging hook 1213 of the needle output element 121 away from the first engaging hole 1112 to release the needle output element 121 that is located at the starting position of the operation stroke L from the locked state. Meanwhile, the contact end cap 113 pushes the abutting sheet to activate the confirming switch 63. The confirming switch 63 sends a confirming signal to the control unit 60 to switch the control unit 60 of the drug injection system to the state of preparation for injection (i.e. standby mode).

Then, the needle output element 121 is pressed. When the needle output element 121 is pressed to move along the operation stroke L, the toothed driving portion 1212 of the needle output element 121 drives the gear portion 1221 of the rotating element 122 with the forward rotating rack segment 12121 and the reverse rotating rack segment 12122 successively, so that the rotating element 122 is driven to rotate in the forward direction and then in the reverse direction by the needle output element 121. Furthermore, the rotating element 122 drives the needle seat 124 that is connected to the piercing assembly 13 to move downward and then upward. With the downward movement of the needle seat 124, the piercing assembly 13 that is formed by combining the conduit element 132 and the piercing needle 131 is driven to pierce downward through the sealed portion 1234 on the bottom of the needle seat plug 123 to be in the needle output state and pierce into the human body. Afterwards, in the needle retraction state, the piercing needle 131 moves upward along with the needle seat 124 to retract the piercing needle 131 and the conduit element 132 is detained. Part of the conduit element 132 protrudes out of the sealed portion 1234 and is disposed outside the casing 11. The conduit element 132 remains piercing in the human body. Meanwhile, as shown in Figs. 25 to 27 and 22, the engaging hook 1213 of the needle output element 121 that is moved to the end position of the operation stroke L is able to engage in the second engaging hole 111 of the base 12 to keep the needle output element 121 in the locked state without pressing the needle output element 121. On the other hand, when the needle output element 121 is pressed to the end position, the activating switch 62 is activate to start an infusion state. The control unit 60, which was originally in the state of preparation for injection (i.e. standby mode), receives the signal from the activating switch 62 and activates the motor 70 and drives the piston rod 24 of the adapting mechanism 20 to move at a predetermined speed through the transmission assembly 80, so as to draw the medicinal liquid in the drug storage module 2 to flow through the adapting mechanism 20 to the liquid injection room 1232 of the needle seat plug123 and to inject the medicinal liquid into the human body from the liquid injection room 1232 through the conduit element 132. Every time the drive element 83 that is connected to the piston rod 24 performs the push-injection movement, the counting element 64 can be activated. The control unit 60 determines the timing to stop the motor 70 according to the received injection signal from the counting element 64. When the control unit 60 receives the injection signal generated by the counting element 64 while being activated, the control unit 60 starts timing. After waiting for predetermined seconds, the transmission assembly 80 drives the piston rod 24 of the adapting mechanism 20 to perform the next injection stroke.

As shown in Figs. 11 and 20 to 24, in the forgoing description, when the piston rod 24 is driven to move backward by the driving module 3, the piston chamber 230 in the piston barrel 23 is under negative pressure, the one-way output valve portion 222 of the flow channel switching valve 22 of the adapting mechanism 20 is closed, and the one-way input valve portion 221 is opened. Thus, the medicinal liquid in the drug storage module 2 flows into the piston chamber 230 through the container connector 32 and the medicinal liquid inflow channel 211 and the one-way input valve portion 221 of the adapting mechanism 20. Then when the piston rod 24 is driven to move forward by the driving module 3, the medicinal liquid in the piston chamber 230 is pushed and under positive pressure, the one-way output valve portion 222 of the flow channel switching valve 22 of the adapting mechanism 20 is opened, and the one-way input valve portion 221 is closed. Thus, the medicinal liquid in the piston chamber 230 flows into the liquid injection room 1232 of the needle seat plug 123 of the needle output mechanism 10 through the medicinal liquid outflow channel 212 and the one-way output valve portion 222 of the adapting mechanism 20, and then is injected into the human body from the liquid injection room 1232 through the conduit element 132.

As shown in Figs. 11 and 20 to 24, after the control unit 60 determines that the target total infusion volume of the drug has been reached according to the number of the injection signals sent by the counting element 64, the motor 70 is stopped and the injection is ended. Then the user is able to remove the drug injection system. In this way, the contact end cap 113 protrudes out of the casing 11 due to the resilient force of the resilient element 114 and is moved away from the confirming switch 63, so that the confirming switch 63 sends a signal to the control unit 60 to be switched to an idle state. Moreover, the conduit element 132 is disposed in the contact end cap 113 that protrudes out of the casing 11. With the contact end cap 113 sleeving the conduit element 132, the safety during use is ensured.

In addition, the control unit 60 of the drug injection system can also provide warning functions with light and sound and safety mechanisms through its built-in control program in cooperation with the confirming switch 63, the indication light 65 including the first light-emitting element (such as the blue light-emitting element) and the second light-emitting element (such as the red light-emitting element) that emit light of different colors, and the sound generator 66, as explained in detail below.

For the purpose of indicating usage status of the drug injection system and warning, the indication light 65 is under the control of the control unit 60. The indication light 65 is able to cause the first light-emitting element (such as the blue light-emitting element) and the second light-emitting element (such as the red light-emitting element) that emit light of different colors to respectively emit or turn off light according to different states.

When the insulating sheet 55 is drawn out, the power supply unit 53 supplies the electrical power to the control unit 60 of the driving module 3 through the electrical connector 54. If the control unit 60 determines that the electrical power of the power supply unit 53 is greater than a predetermined value or ratio, an idle state is entered normally. Then the first light-emitting element (the blue light-emitting element) and the second light-emitting element (the red light-emitting element) of the indication light 65 is controlled to light up and the sound generator 66 is controlled to make a prompting sound. Afterwards, the second light-emitting element (the red light-emitting element) is controlled to be turned off and the first light-emitting element (the blue light-emitting element) remains lighting. If the control unit 60 determines that the electrical power of the power supply unit 53 is less than a predetermined value or ratio, for example, the electrical power is less than 25% of the original stored electrical power, the second light-emitting element (the red light-emitting element) of the indication light 65 is controlled to light up and enters a flashing cycle and the sound generator 66 is controlled to make a warning sound. Afterwards, the second light-emitting element (the red light-emitting element) is controlled to be turned off, and the drug injection system automatically shuts down and locks due to insufficient electrical power of the power supply unit 53 and can no longer be used for safety concern.

When the drug injection system is attached to the human body, the contact end cap 113 pushes the abutting sheet 56 to activate the confirming switch 63. The confirming switch 63 sends a confirming signal to the control unit 60 to switch the control unit 60 of the drug injection system to the state of preparation for injection (i.e. standby mode). In the standby mode, if the attachment becomes loose, the contact end cap 113 is unable to keep activating the confirming switch 63. Then the idle state would be resumed under control of the control unit 60. If the drug injection system is re-attached to the human body within a specific period of time (for example: 10 seconds) to allow the contact end cap 113 to activate the confirming switch 63 again, the standby mode can be resumed. If the specific period of time is exceeded and the contact end cap 113 is still not restored to activate the confirming switch 63, the drug injection system would be automatically shut down and locked under the control of the control unit 60.

When the drug injection system is accurately attached to the human body and the needle output element 121 is pressed to the end position, the activating switch 62 is activated to enter the infusion state. In the infusion state, if the attachment becomes loose, the contact end cap 113 is unable to keep activating the confirming switch 63. Then the second light-emitting element (the red light-emitting element) of the indication light 65 is lighted up and the sound generator 66 makes the warning sound under the control of the control unit 60. If the drug injection system is re-attached to the human body within a specific period of time (for example: 5 seconds) to allow the contact end cap 113 to activate the confirming switch 63 again, the infusion state can be resumed to continue to infuse liquid. If the specific period of time is exceeded and the contact end cap 113 is still not restored to activate the confirming switch 63, the drug injection system would be automatically shut down and locked under the control of the control unit 60 and can no longer be used for safety concern.

When infusion of the liquid is completed normally, the drug injection system enters the idle state. Under the control of the control unit 60, the first light-emitting element (blue light-emitting element) of the indication light 65 continues to light up for a specific period of time and then is turned off, and the drug injection system is locked.

As described above, through built-in control program at the control unit 60 and systematic combination of the confirming switch 63, the indication light 65 and the sound generator 66, the drug injection system of the present invention provides warning functions with light and sound and safety mechanisms during the use of the drug injection system to ensure that the drug injection system of the present invention is safety for use.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A needle output mechanism (10), **characterized in** including:
a casing (11);
a needle output operation assembly (12) assembled in the casing (11) and the needle output operation assembly (12) including a needle output element (121), a rotating element (122) and a needle seat (124); the needle output element (121) movably mounted in the casing (11); the rotating element (122) rotatably mounted in the casing (11) and the rotating element (122) being able to be driven by the needle output element (121) to rotate; the needle seat (124) movably mounted in the rotating element (122) and the needle seat (124) being able to be driven by the rotating element (122) to move be between a first position and a second position; and
a piercing assembly (13) assembled in the casing (11) and the piercing assembly (13) including a piercing needle (131) and a conduit element (132); the piercing needle (131) the piercing assembly (13) connected with the needle seat (124); the conduit element (132) being able to be combined with the piercing needle (131); wherein in a needle output state, the piercing assembly (13) that is formed by combining the conduit element (132) and the piercing needle (131) is driven by the needle output operation assembly (12) to cause at least part of the piercing assembly (13) to protrude out of the casing (11); wherein in a needle retraction state, the piercing needle (131) moves upward along with the needle seat (124) to retract the piercing needle (131).

2. The needle output mechanism (10) as claimed in claim 1, **characterized in that**: the needle output element (121) is mounted in the casing (11) to move linearly; the rotating element (122) is mounted in the casing (11) and is able to be driven by the needle output element (121) to rotate forwardly or reversely.

3. The needle output mechanism (10) as claimed in claim 1, **characterized in that**: the needle output operation assembly (12) further includes a needle seat plug (123) and the needle seat plug (123) is mounted in the casing (11); the piercing needle (131) protrudes into the needle seat plug (123); the conduit element (132) is disposed inside the needle seat plug (123) and is sleeved around the piercing needle (131); the needle seat (124) is able to be driven by the rotating element (122) to move between the rotating element (122) and the needle seat plug (123).

4. The needle output mechanism (10) as claimed in claim 3, **characterized in that**: the needle seat plug (123) has an accommodating room (1231), a liquid injection room (1232) disposed at a lower end of the accommodating room (1231), a connecting port (1233) disposed on a side of the liquid injection room (1232) and communicating with the liquid injection room (1232), and a sealed portion (1234) disposed at a lower end of the liquid injection room (1232) and exposed on a back surface of the casing (11); wherein in the needle output state, the piercing assembly (13) pierces through the sealed portion (1234) on a bottom of the needle seat plug (123); wherein in the needle retraction state, part of the conduit element (132) protrudes out of the sealed portion (1234).

5. The needle output mechanism (10) as claimed in claim 1, **characterized in that**: the rotating element (122) has a threaded hole (1220) formed therein; a threaded portion (1241) is provided on the needle seat (124) and engages with the threaded hole (1220) of the rotating element (122); a gear portion (1221) is provided on an upper end of the rotating element (122); the needle output element (121) has an elongated rail groove (1211) and a toothed driving portion (1212) provided in the elongated rail groove (1211), and a length of the elongated rail groove (1211) is defined as an operation stroke (L); the toothed driving portion (1212) includes a forward rotating rack segment (12121) and a reverse rotating rack segment (12122); the forward rotating rack segment (12121) and the reverse rotating rack segment (12122) are two different sections respectively disposed along the elongated rail groove (1211) and are disposed on two opposite sides; the gear portion (1221) of the rotating element (122) is disposed in the elongated rail groove (1211); wherein with movement of the needle output element (121) along the operation stroke (L), the gear portion (1221) of the rotating element (122) is able to mesh with either the forward rotating rack segment (12121) or the reverse rotating rack segment (12122) respectively, such that the rotating element (122) is able to be driven to rotate in a forward direction and in a reverse direction.

6. The needle output mechanism (10) as claimed in any one of claims 1 to 5, **characterized in that**: the casing (11) includes a base (111) and an outer casing (112) sleeved around the base (111); a back side of the outer casing (112) is mounted with a contact end cap (113) that is able to travel within a movement stroke (S); a resilient element (114) is disposed between the outer casing (112) and the contact end cap (113); the needle output element (121) is mounted in the base (111) and is able to move within a distance of an operation stroke (L); the needle output element (121) is able to be locked in the base (111) at a starting position and an end position of the operation stroke (L) respectively to be in a locked state; resilient force of the resilient element (114) acts on the contact end cap (113), causing part of the contact end cap (113) to protrude from a back side of the outer casing (112); the contact end cap (113) is able to be forced to enter the outer casing (112) to release the needle output element (121) that is located at the starting position of the operation stroke (L) from the locked state.

7. The needle output mechanism (10) as claimed in claim 6, **characterized in that**: the base (111) has a first engaging hole (1111) disposed on the starting position of the operation stroke (L) and a second engaging hole (1112) disposed on the end position of the operation stroke (L); an engaging hook (1213) is provided on a rear end of the needle output element (121) and the engaging hook (1213) is able to selectively engage in the first engaging hole (1111) or the second engaging hole (1112); the contact end cap (113) has an unlocking pushing portion (1131); wherein when the contact end cap (113) is forced to enter the outer casing (112), the unlocking pushing portion (1131) is able to push the engaging hook (1213) of the needle output element (121) to disengage from the first engaging hole (1111) to release the needle output element (121) that is locked from the locked state.

8. An injection channel module (1), **characterized in** including:
a needle output mechanism (10) as claimed in any one of claims 1 to 5; and
an adapting mechanism (20) assembled to a side of the needle output mechanism (10) and the adapting mechanism (20) including an adapting seat (21), a flow channel switching valve (22), a piston barrel (23) and a piston rod (24); the adapting seat (21) assembled in the needle output mechanism (10); the flow channel switching valve (22) and the piston barrel (23) both mounted in the adapting seat (21); the adapting seat (21) having a medicinal liquid inflow channel (211) and a medicinal liquid outflow channel (212) that communicate with the piston barrel (23); the flow channel switching valve (22) communicating with the connecting port (1233) of the needle output mechanism (10) through the medicinal liquid outflow channel (212) of the adapting seat (21); the piston rod (24) linearly movably assembled in the piston barrel (23).

9. The injection channel module (1) as claimed in claim 8, **characterized in that**: the flow channel switching valve (22) includes a one-way input valve portion (221) and a one-way output valve portion (222); two ends of the piston barrel (23) are respectively a connecting end (231) and a barrel opening end (232); the connecting end (231) is connected to the flow channel switching valve (22) and the barrel opening end (232) protrudes out of the adapting seat (21); the piston barrel (23) has a piston chamber (230) formed therein; the connecting end (231) has a medicinal liquid inlet (2311) and a medicinal liquid outlet (2312) communicating with the piston chamber (230); the medicinal liquid inlet (2311) is connected to the one-way input valve portion (221) and the medicinal liquid outlet (2312) is connected to the one-way output valve portion (222); the piston rod (24) is able to be driven to move linearly in the piston chamber (230) of the piston barrel (23) to draw medicinal liquid into the piston chamber (230) through the medicinal liquid inflow channel (211) and the one-way input valve portion (221) in one way and output the medicinal liquid to the medicinal liquid outflow channel (212) through the one-way output valve portion (222) in one way.

10. A drug injection system, **characterized in** comprising:
an injection channel module (1) including a needle output mechanism (10) as claimed in any one of claims 1 to 5 and an adapting mechanism (20), wherein the adapting mechanism (20) is assembled to a side of the needle output mechanism (10) and the adapting mechanism (20) includes an adapting seat (21), a flow channel switching valve (22), a piston barrel (23) and a piston rod (24); the adapting seat (21) is assembled in the needle output mechanism (10); the flow channel switching valve (22) and the piston barrel (23) are both mounted in the adapting seat (21); the adapting seat (21) has a medicinal liquid inflow channel (211) and a medicinal liquid outflow channel (212) that communicate with the piston barrel (23); the flow channel switching valve (22) communicates with the connecting port (1233) of the needle output mechanism (10) through the medicinal liquid outflow channel (212) of the adapting seat (21); the piston rod (24) is linearly movably assembled in the piston barrel (23);
a drug storage module (2) including a medicinal liquid storage container (30) and an outer covering (40); the medicinal liquid storage container (30) including a container body (31) filled with medicinal liquid and a container connector (32) assembled to the container body (31); the container connector (32) connected to the medicinal liquid inflow channel (211) of the adapting mechanism (20) of the injection channel module (1); the outer covering (40) being able to be sleeved around the medicinal liquid storage container (30) and connected with the casing (11) of the needle output module; and
a driving module (3) detachably assembled to the injection channel module (1) and being able to drive the piston rod (24) of the adapting mechanism (20) to move.

11. The drug injection system as claimed in claim 10, **characterized in that**: the container connector (32) includes a connector body; the connector body includes a container connecting end (3211), an infusion connecting end (3212) and an infusion end portion (3213); the infusion channel extends from the container connecting end (3211) to the infusion connecting end (3212) and is connected with the container body (31) through the container connecting end (3211); the infusion connecting end (3212) is connected to the medicinal liquid inflow channel (211) of the adapting mechanism (20); the infusion end portion (3213) has an infusion branch channel (32131) communicating with the infusion channel and an infusion plug (3214) mounted in the infusion branch channel (32131) and sealing the infusion branch channel (32131); the infusion plug (3214) has a plug end (32141) exposed outside the connector body and the plug end (32141) is exposed on a back surface of the outer covering (40).

12. The drug injection system as claimed in claim 10, **characterized in that**: the casing (11) of the needle output mechanism (10) includes a base (111) and an outer casing (112) sleeved around the base (111); a back side of the outer casing (112) is mounted with a contact end cap (113) that is able to travel within a movement stroke (S); a resilient element (114) is disposed between the outer casing (112) and the contact end cap (113); the needle output element (121) is mounted in the base (111) and is able to move within a distance of an operation stroke (L); the needle output element (121) is able to be locked in the base (111) at a starting position and an end position of the operation stroke (L) respectively to be in a locked state; resilient force of the resilient element (114) acts on the contact end cap (113), causing part of the contact end cap (113) to protrude from a back side of the outer casing (112); the contact end cap (113) is able to be forced to enter the outer casing (112) to release the needle output element (121) that is located at the starting position of the operation stroke (L) from the locked state;
the flow channel switching valve (22) includes a one-way input valve portion (221) and a one-way output valve portion (222); two ends of the piston barrel (23) are respectively a connecting end (231) and a barrel opening end (232); the connecting end (231) is connected to the flow channel switching valve (22) and the barrel opening end (232) protrudes out of the adapting seat (21); the piston barrel (23) has a piston chamber (230) formed therein; the connecting end (231) has a medicinal liquid inlet (2311) and a medicinal liquid outlet (2312) communicating with the piston chamber (230); the medicinal liquid inlet (2311) is connected to the one-way input valve portion (221) and the medicinal liquid outlet (2312) is connected to the one-way output valve portion (222); the piston rod (24) is able to be driven to move linearly in the piston chamber (230) of the piston barrel (23) to draw medicinal liquid into the piston chamber (230) through the medicinal liquid inflow channel (211) and the one-way input valve portion (221) in one way and output the medicinal liquid to the medicinal liquid outflow channel (212) through the one-way output valve portion (222) in one way;
the driving module (3) includes a housing (50), a control unit (60), a motor (70) with a central shaft, and a transmission assembly (80); the control unit (60), the motor (70) and the transmission assembly (80) are all mounted in the housing (50); the motor (70) is electrically connected to and is controlled by the control unit (60); the transmission assembly (80) includes a driving wheel (81), a connecting rod (82) and a drive element (83); the driving wheel (81) is assembled to the central shaft of the motor (70); an end of the connecting rod (82) is eccentrically connected pivotally to the driving wheel (81) and another end of the connecting rod (82) is connected to the drive element (83) and is detachably connected to a distal end of the piston rod (24) through the drive element (83); a movement direction of the connecting rod (82) and the drive element (83) is parallel with a central axis of the piston rod (24).

13. The drug injection system as claimed in claim 12, **characterized in that**: the housing (50) includes a housing body (51) and a housing cover (52) mounted on the housing body (51); a power supply unit (53) and two electrical connectors (54) are able to be mounted in the housing (50); the power supply unit (53) is able to be electrically connected to the control unit (60) through the two electrical connectors (54); an insulating sheet (55) is provided between an end of the power supply unit (53) and one of the electrical connectors (54) that corresponds in position thereto for insulation and isolation; the insulating sheet (55) protrudes out of the housing (50).

14. The drug injection system as claimed in claim 12, **characterized in that**: the driving module (3) includes an activating switch (62), a confirming switch (63) and a counting element (64); the activating switch (62), the confirming switch (63) and the counting element (64) are all electrically connected to the control unit (60); the activating switch (62) is mounted on the housing (50) and is disposed at the end position of the operation stroke (L) of the needle output element (121); wherein when the needle output element (121) is pressed to the end position, the activating switch (62) is activated;
the confirming switch (63) is mounted in the housing (50) and is disposed at an end position of the movement stroke (S) of the contact end cap (113); wherein when the contact end cap (113) is pressed to the end position, the confirming switch (63) is activated;
the counting element (64) is mounted in the housing (50) and is disposed at a movement path of the drive element (83); wherein when the drive element (83) is moved, the counting element (64) is activated.
